(19) Europäisches Patentamt
European Patent Office
Office européen des brevets

(11) EP 4 073 808 B1

# (12) EUROPEAN PATENT SPECIFICATION

(45) Date of publication and mention of the grant of the patent:
**15.04.2026 Bulletin 2026/16**

(21) Application number: **20819789.7**

(22) Date of filing: **09.12.2020**

(51) International Patent Classification (IPC):
***G16B 30/20*** *(2019.01)*

(52) Cooperative Patent Classification (CPC):
**G16B 30/00**

(86) International application number:
**PCT/EP2020/085368**

(87) International publication number:
**WO 2021/116224 (17.06.2021 Gazette 2021/24)**

## (54) INDEX SEQUENCES FOR MULTIPLEX PARALLEL SEQUENCING

INDEXSEQUENZEN ZUR MULTIPLEX-PARALLELSEQUENZIERUNG

SÉQUENCES D'INDICE DE SÉQUENÇAGE PARALLÈLE DE MULTIPLEXAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.12.2019 EP 19214355**

(43) Date of publication of application:
**19.10.2022 Bulletin 2022/42**

(73) Proprietor: **Lexogen GmbH**
**1030 Vienna (AT)**

(72) Inventors:
- **MOLDASCHL, Michael**
  **3483 Wagram am Wagram (AT)**
- **TÜRK, Andreas**
  **1020 Wien (AT)**
- **REDA, Torsten**
  **1140 Wien (AT)**
- **SCHAUPPER, Mira Valentina**
  **1050 Wien (AT)**

(74) Representative: **SONN Patentanwälte GmbH & Co KG**
**Riemergasse 14**
**1010 Wien (AT)**

(56) References cited:
**WO-A1-2018/204423 US-A1- 2019 085 384**

- **TILO BUSCHMANN ET AL: "Levenshtein error-correcting barcodes for multiplexed DNA sequencing", BMC BIOINFORMATICS, vol. 14, no. 1, 1 January 2013 (2013-01-01), pages 272, XP055194297, ISSN: 1471-2105, DOI: 10.1186/1471-2105-14-272**
- **PANU SOMERVUO ET AL: "BARCOSEL: a tool for selecting an optimal barcode set for high-throughput sequencing", BMC BIOINFORMATICS, BIOMED CENTRAL LTD, LONDON, UK, vol. 19, no. 1, 5 July 2018 (2018-07-05), pages 1 - 6, XP021258232, DOI: 10.1186/S12859-018-2262-7**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### Field of the invention

The present invention relates to next generation sequencing and oligonucleotide identification in multiplex methods.

### Background of the invention

Index sequences, also referred to as barcodes, are commonly used as short sequences of nucleotides that are added to the fragments in a library, such that fragments from one sample are associated with a unique non-empty set of barcodes. This allows multiple samples to be mixed and sequenced together decreasing sequencing costs and increasing throughput (parallel sequencing or multiplexing). This procedure is visualized in Figure 1. The left side of Figure 1 shows three samples (ellipsoids), each containing a collection of fragments (curly lines) . During multiplexing, barcodes BC1, BC2 and BC3 are added to the fragments in sample one, two and three, respectively, and barcoded fragments are mixed together. In Figure 1, therefore, the non-empty set of barcodes associated with a sample consists of a single barcode. This is the most common situation in multiplexing, resulting in a one-to-one relationship between barcodes and samples. After sequencing the multiplexed library, the barcode sequence of each fragment is investigated. If the sequence corresponds to the nucleotide sequence of barcode BC1, BC2 or BC3 the non-barcode sequence of the fragment is assigned to sample one, two and three, respectively. This process of assigning fragment sequences to samples according to their associated barcode sequence is called demultiplexing.

Barcode synthesis, library preparation and sequencing can introduce errors in the barcode sequence and demultiplexing might therefore result in an incorrect assignment of fragments to samples. To avoid contamination of samples with fragment sequences from other samples, barcodes are usually designed to minimize the chance of transforming into each other. This can be done by maximizing the number of changes needed to transform one barcode into another, or in other words by maximizing the inter-barcode distance. Since the achievable inter-barcode distance increases with decreasing number of samples, the barcode set for an experiment should be optimized with respect to the number of samples in the experiment. Inter-barcode distance can, further, be increased by increasing the barcode length. This, however, comes at the cost of a reduction in the length of the sequenced fragment, since the combined number of sequenced nucleotides for barcode and fragment is limited. Hence, the barcode length for an experiment should be chosen such that the required level of cross-contamination is achieved without unnecessarily sacrificing fragment sequence length.

If the inter-barcode distance is large enough, minor errors can still lead to an assignment that is probably correct. These are called error-correcting barcodes and usually use a distance estimation method that closely resembles the amount of nucleotide changes that can happen in the physical sample (see e.g. Buschmann et al. [1], Hawkins et al. [3], WO 2016/018960 A1). Other approaches that address other problems that could prevent proper assignment, such as index hopping, are the use of dual indexes (see MacConaill [5] and WO 2018/136248 A1).

WO 2018/204423 A1 discloses color-balancing of index sequences by pairing A and C with G and T (or U).

WO 2011/100617 A1 discloses index sequences that do not have 4 or more contiguous identical subunits.

US 2019/085384 A discloses a set of sequencing adapters, wherein each adapter comprises a variable-length non-random unique molecular indexe ("vNRUMI") to identify individual nucleic acid molecules for multiplex parallel sequencing.

### Summary of the invention

Despite various attempts of improving barcodes, there remains a need to provide improved index sequence oligonucleotides that have an optimal distinguishability that allows assignment even in the event of errors. These barcodes should maximize this distinguishability for a sample at hand as used by a practitioner but still allow a compromise with efficiency considering the increased effort and cost for each nucleotide that has to be sequenced.

The invention provides a computer-implemented method of generating a set of oligonucleotides comprising a plurality of subsets of oligonucleotides with a subset of index sequences comprising the steps of generating a first subset of oligonucleotides with index sequences with a first sequence distance to each other within the first subset, wherein a sequence distance is a quantified amount of sequence changes that transforms one sequence into another or a monotonically decreasing function of a probability of sequence changes that transforms one sequence into another, generating a second subset by including the first subset and adding further oligonucleotides with index sequences with a second sequence distance to each other within the second subset, which second sequence distance is a lower sequence distance than the first sequence distance, whereby the second subset comprises oligonucleotides with index sequences that are not part of the first subset.

The method can generate a disclosed set of oligonucleotides comprising index sequences and wherein the set comprises a plurality of subsets of oligonucleotides with different index sequences, wherein the index sequences of a subset of oligonucleotides differ at least by a non-zero number of sequence changes from each other; and wherein the set comprises at least 2 hierarchical tiers of subsets, wherein index sequences of a higher tier subset are members of a lower tier subset, and wherein the index sequences of a lower tier subset differ by a lower minimum number of sequence changes from each other than the index sequences of a higher tier subset; and wherein the oligonucleotides are assigned to one or more subsets.

The invention further provides a computer-implemented method of assigning sequencing reads to a sample of oligonucleotides comprising the steps of

a) obtaining sample oligonucleotides from a plurality of samples,
b) generating a set of oligonucleotides according to the invention and selecting a subset of oligonucleotide index sequences from the set of oligonucleotides, wherein a subset is selected over another subset based on a higher sequence distance of the index sequences to each other within the selected subset; wherein a sequence distance is a quantified amount of sequence changes that transforms one sequence into another or a monotonically decreasing function of a probability of sequence changes that transforms one sequence into another, and wherein the selected subset has at least as many different index sequences as the number of samples of step a), c) adding index sequences from said subset to each sample oligonucleotide wherein the index sequences are indicative of the sample, d) determining the sequence of the sample oligonucleotides or fragments of sample oligonucleotides and determining the index sequence, e) assigning an obtained read sequence to a sample based on the determined index sequence or based on the index sequence which has the lowest sequence distance to the determined index sequence, wherein if two or more index sequences have the same lowest distance then said obtained read is discarded; wherein optionally the sequence distance does not exceed a pre-set criterion value.

The following detailed description and preferred embodiments apply to all disclosed aspects and can be combined with each other without restriction, except were explicitly indicated. E.g. the disclosed set can be obtainable by the method of generation; the set can be suitable for the method of assigning sequencing reads. Embodiments and aspects of the invention are defined in the claims.

**Figures**

Figure 1: Multiplexing, sequencing and demultiplexing. Fragments (curly lines) in three samples (ellipsoids) are barcoded with index seqeunces BC1, BC2 and BC3.

Figure 2: Nested barcode sets. Smaller index sequence sets (subsets of higher tier) are contained in larger index sequence sets (subsets of lower tier). Increasing barcode set size reduces inter-barcode distance.

Figure 3: Nested barcode sequences. Extending index sequences increases inter-barcode distance and retains the nested structure of index sequence sets.

Figure 4: Schematic of a dynamic programming algorithm for calculation of Levenshtein distances.

Figure 5: Schematic of a reverse probability calculation.

Figure 6: Distribution of $B_1$,$B_2$,$B_3$,$B_4$ on a 8x12 well-plate.

Figure 7: read and index sequence layout for dual indexing (i7/i5).

Figure 8: Positional nucleotide distribution for $B_1$ with $|B_1| = 4$.

Figure 9: Positional nucleotide distribution for $B_2$ with $|B_2| = 8$.

Figure 10: Positional nucleotide distribution for $B_3$ with $|B_3| = 16$.

Figure 11: Positional nucleotide distribution for $B_4$ with $|B_4| = 24$.

Figure 12: Count matrix for dual-index experiment measuring synthesis provider dependent cross-contamination.

## Detailed description of the invention

As used herein, the word "barcode" refers to a "index sequence", which is a sequence of nucleotides that is capable of and is used to identify sequences (usually in oligonucleotides or sequencing reads thereof) that are labelled with these index sequences. In the sets and subsets, these index sequences are included into oligonucleotides and thus the oligonucleotides have a nucleotide sequence of said index sequence. The oligonucleotides may comprise further nucleotides or not. Usually the oligonucleotides are used to label other nucleic acids of a sample by attachment and thus the resulting oligonucleotide has more nucleic acids. It is also possible to label other moieties, such as proteins, such as antibodies or enzymes, or beads or particles, such as nanoparticles, or cells or chemical compounds, such as drugs, by attachment thereto.

Disclosed is a set of oligonucleotides comprising index sequences and wherein the set comprises a plurality of subsets of oligonucleotides with different index sequences. The index sequences of a subset of oligonucleotides differ at least by a non-zero number of sequence changes from each other. Such sequence changes can be estimated by a sequence distance as will be described in more detail below. Using the sequence distance terminology one can also state that the sequence distance of the index sequences is non-zero. It can be 1 or more in distances that are indicated as integers or a non-zero fraction or function of a fraction (such as sequence change probabilities) . The set comprises at least 2 (i.e. 2 or more) hierarchical tiers of subsets, wherein index sequences of a higher tier subset are members of a lower tier subset. This means that the set comprises a first subset and at least one further (second or more) subset that contains the members of the first subset. The first subset is considered as the subset of the higher tier and when "first" represents the first of all subsets, even highest subset. This means that lower tier subsets contain more members (sequence indexes) than higher tier subsets. By including more members, the distance between all these members (minimum distance or smallest distance) decreases, in case the index sequence length remains the same. Accordingly, in the inventive set, the index sequences of a lower tier subset differ by a lower minimum number of sequence changes from each other than the index sequences of a higher tier subset.

"Minimum number of sequence changes" refers to the lowest number of sequence changes that is present for all possible sequence changes between any two members of a subset.

The oligonucleotides of the set are assigned to one or more subsets. This means that a user knows to which subset each index sequence (or oligonucleotide) belongs to. Such an assignment can be done physically, e.g. by placing the oligonucleotides into containers that are labelled or ordered according to the subset assignment.

The subset structure is also referred to as "nested sets" as one subset is nested in (or a member of) another subset. E.g. the index sequences of a first subset can be contained in said first subset and also in a second subset, to which also further index sequences belong that are not found in the first subset.

This nested hierarchy of subsets allows the provision of several subsets of index sequences that have different sizes. "Size of a subset" is understood as the amount of different index sequences in said subset. These subsets of different sizes make it possible to avoid multiple physical barcode sets for different applications thereof dependent on the need for different sizes. A practitioner who uses the inventive nested sets can choose from a range of subsets to fit the practitioner's size requirement, e.g. the number of samples that need to be individually labelled by the index sequences. By choosing a higher tier subset - as possible dependent on the size requirement due to the number of samples - the practitioner can optimize the distance between the index sequences and thus increase assignment quality of the labelled objects, such as reads or fragment sequences, to a sample.

Assignment quality in essence means confidentiality of an assignment and the possibility to assign a determined sequence of an index to a sample even if that determined sequence is not identical to the index sequences of said sample, e.g. by assigning said diverging determined sequence to a sample if it has the lowest distance to the correct index sequence of that sample (error correction) as compared to other index sequences of other samples. This type of error correction is known in the art - see ref. [1]. "Index sequence of that sample" means the error free index sequence that has been assigned to a sample by the practitioner, such as by binding oligonucleotides with the index sequence to sample nucleic acids. Assignment quality is thus a property to evaluate miss-assignment and cross-contamination.

A further aspect that affects assignment quality - besides the provision of subset sizes to fit the sample needs - is the index sequence length. Some simple samples or reliable measurement set-ups only require a low distance between index sequences while other more complex samples or error-prone measurement set-ups need larger distances. Since the determination of each index sequence nucleotide increases costs (especially in large scale multiplex methods), it is therefore desirable to measure only as many nucleotides of the index sequence as needed or as acceptable for a given application. To also accommodate the need for a flexible selection of the index sequence lengths, index sequences are also useful when only a part of the index sequence or truncated index sequence is used for assignment. To achieve this goal, these truncated index sequences are adjusted within a subset so that a reliable distance is maintained.

The truncated index sequences are parts of the index sequences that are suitable to maintain a desired distance

from each other truncated index sequence of the same subset. This property of being a part of a larger sequence is also referred to as a "nested sequence", referring to a sequence within a sequence. This should not be confused with the nested subsets mentioned above, which refers to subsets within other subsets.

The truncated, nested, index sequence properties allow the use of the entire index sequence in experiments that could be satisfied with shorter index sequences as well as the use in experiments that need longer index sequences. A practitioner thus only needs one such set that is universally useful. In practice, for an experiment, the user usually selects barcodes from the smallest of the nested sets larger than the number of samples, and sequences as many nucleotides of the barcodes (index sequences) as necessary to achieve the required (low) level of cross-contamination. The nested barcode sets obtain an increase in inter-barcode distance for smaller sets and for longer sequences. This guarantees that the user will always select the optimal configuration among all possible combinations of nested sets and sequences.

Accordingly, the index sequences of a subset may contain each a truncated index sequence and the truncated index sequences of at least one subset differ at least by a non-zero number of sequence changes from each other truncated index sequence (inter-barcode distance) within said subset.

Preferably, the minimum number of sequence changes between truncated index sequences of a subset is larger than the minimum number of sequence changes of the index sequences in the subset minus the difference between the length of the index sequences and the truncated index sequences or in other more general words, preferably the sequence distance (as explained herein) between truncated index sequences of a subset is larger than the sequence distance of the index sequences in the subset minus the difference between the length of the index sequences and the truncated index sequences. This formula essentially means that the nucleotides that are not considered in the index sequence to obtain the truncated index sequence (expressed as length difference) shall not be strong determinants of the sequence distance, meaning that the remaining nucleotides in the truncated index sequences have a strong impact on sequence distance. Usually such a structure within the (nested) index sequence is established beforehand and communicated to the practitioner, so that the practitioner knows which nucleotides shall be determined as truncated index sequence. Preferably the truncated index sequence is composed of continuous nucleotides of the index sequence. Especially preferred, the truncated index sequence comprises the 3’ or 5’ end of the index sequence.

As for nested subsets, the concept on truncated index sequences can be applied multiple times yielding multiple nested index sequences. This means that more than one tier of truncation is possible. In case of several truncation steps, each truncated index sequence has a certain distance with each other truncated index sequence of the same tier within a subset. There may be 1, 2, 3, 4, 5 or more tiers of tuncated index sequences, of which 2 are preferred since this can be well accomodated in common index sequence lengths.

Of course the nested sequences can be combined with the nested set structure. The tier structure for the subsets remains the same. Thus, the truncated index sequences of a higher tier subset are members of truncated index sequences of a lower tier subset. Due to the differences in subset sizes, the truncated index sequences of a lower tier subset may differ by a lower minimum number of sequence changes from each other than the truncated index sequences of a higher tier subset.

Various methods to determine sequence distance exist as described in the references mentioned above in the background section. Any of these methods can be used. In particular, the sequence changes are preferably selected from nucleotide substitutions, deletions and insertions. The minimum number of sequence changes corresponds to the minimum of these sequence changes that are needed to change any index sequence to another index sequence. Multiple paths to change one sequence to another may exist, whereas the "distance" refers to the shortest paths, i.e. the ones with the least changes (minimum). This may be one path or more than one path when multiple paths have the same minimum distance. A further distance option that can be used to quantify the amount of sequence changes that transforms one sequence into another is a sum of the individual distances of single paths of changes that transform one sequence into another. Such a sum can be used for all paths for a given change. The paths should be direct paths from one sequence to another without detours such as changes that cancel each other out.

Sequence distances described in the art (see background section) are e.g. a Hamming distance, a Levenshtein distance or a Sequence-Levenshtein distance. These distances can be used to quantify the distance or determine the amount or number of sequence changes that transforms one sequence into another. A Hamming distance is in essence a count of substitutions. The Levenshtein distance is calculated using insertions, deletions (together "indels") and substitutions. Preferably a Sequence-Levenshtein distance (ref. [1]) is used. The Sequence-Levenshtein distance is a variant of the Levenshtein distance that also considers indels and substitutions but maintains the index length whenever an insertion or deletion occurs. This means that an insertion and a deletion will be counted at most as one change. A deletion may also result in no change in case if the last nucleotide in a sequence is deleted and the next nucleotide outside the frame that now moves into frame is identical to the deleted nucleotide. Likewise, the insertion of an identical nucleotide at the last nucleotide in the sequence may not be apparent as a change and produce no distance. Contrary thereto, the Levenshtein distance considers a deletion in the context of an oligonucleotide sequence where the index sequence is followed by other nucleotides (such as of an adapter or of the product read) as two changes: one, the removal of the deleted nucleotide and two, the shift of a following nucleotide into the frame of the sequence index since the entire length of sequence index is

compared and this shift is counted as another difference between the compared sequences (see ref. [3], Fig. 1 for differences between Hamming, Levenshtein and Sequence-Levenshtein distances). Other terms for a Sequence-Levenshtein distance are FREE Levenshtein distance or modified Levenshtein distance or fixed-frame Levenshtein distance (ff Levenshtein distance). For example, referring to an example in the supplement of ref. [3], which terms the Sequence-Levenshtein distance "FREE divergence", the sequences TAGA and ACGC have a distance of 3 according to the following changes:

$$TAGA \xrightarrow{ins.} TACG|A \xrightarrow{sub.} TACG|C \xrightarrow{del.} ACGC$$

wherein "ins." is an insertion, "sub." a substitution and "del." a deletion (each also being referred to as "edits" or "changes"); the vertical bars ("|") show the end of the barcode frame, though the truncation step would not happen until after all actual edits. These shifts across the frame length lead to a violation of the triangle inequality. Distance methods that do not consider these shifts out of and into the frame of the index sequence (or truncated index sequence) could result in a distance determination that does not reflect the actual changes that transform one sequence into another. In this example the distance of TAGA and TACG would be 1 (insertion of C); so is the distance between TACG and ACGC (deletion of T with 3'C shifting into frame). However, the distance between TAGA and ACGC is not 1+1=2 but 3 as shown above (violation of triangle inequality) . Here a substitution occurs out of frame which may be counted in some methods of distance determination but not in others. Although both types of distance measurements work as they give a comparable indication of the distance between sequences, some distance estimates take sequence changes outside the frame of the index sequence (or truncated index sequence) which shift into the frame of the index sequence (or truncated index sequence) into account to more closely resemble natural processes that transform one sequence into another (for various reasons, such as insertions, deletions and substitutions during sequencing methods). This would be an additional step to the above-mentioned Hamming distance, Levenshtein distance and Sequence-Levenshtein distance. On the other hand, a Sequence-Levenshtein distance (fixed frame) has procedural benefits and is a preferred method. A possible violation of the triangle inequality (meaning the sum of partial distances does not necessarily equate to the full distance) is then usually considered during error correction steps. Another sequence out of frame of the index sequence that can be considered similar to the frame-shifted nucleotides of the index sequence itself are nucleotides or sequences that follow the index sequence. These may be known, such as in the case of an adapter sequence that follows the index sequence.

Generally, the sequence changes can be quantified as sequence distance which is the amount of nucleotide changes or a probability of changes. Each possible change can either be counted as an integer or as its probability. Such a probability may be platform- dependent or a pre-set probability, e.g. from average values may be used. For example, a probability may be inferred from natural mutation rates, such as they are occurring in a sequencer. For example, probabilities for substitutions, insertions and deletions may be 0.002, 0.00002 and 0.0005, respectively in this order.

The probability of changes can be a maximum or a sum of probabilities. In some cases, several series of changes (also referred to as "paths") can lead to the transformation of one (index) sequence into another. In such a case, the path with the highest probability (maximum) can provide a suitable estimate as the sequence distance. Alternatively, the probabilities of several paths can be added to provide a sum of probabilities, which is also a suitable estimate for use as sequence distance. Preferably a sum of probabilities of nucleotide changes that transform one sequence to another is used.

Of note is that the reciprocity between a comparison of probabilities and of an integer count of sequence changes is reversed, whereas a high count of sequence changes equates to a large distance; it is a low probability that corelates with a large distance (and a high probability correlates with a small distance). Accordingly, referring to the relationship of tiers as mentioned above, the index sequences of a lower tier subset differ by a higher probability of sequence changes from each other than the index sequences of a higher tier subset. Also, the truncated index sequences of a lower tier subset can differ by a higher probability of sequence changes than the truncated index sequences of a higher tier subset.

Of course, to maintain the same direction of the relationship (higher-higher; lower-lower), it is possible to use a function of the probability that reverses the order or directionality of the probability. Such functions are monotonically decreasing functions of the probability. Of course this is just another representation of a probability and the relationships of the underlying probabilities (or averages or sums) remain the same. Nevertheless, in preferred embodiments, the probability of changes is quantified by a monotonically decreasing function of a probability. Such a function is for example a negative logarithm or a negative probability (changing its sign, order or directionality), such as in 1-P (with P being a probability, including an average or maximum as mentioned above). Preferably, the probability is estimated as such a monotonically decreasing function by a maximum or a sum of probabilities, preferably a sum of probabilities, of nucleotide changes that transform one sequence to another. Such nucleotide changes can be a series of changes if more than one change is needed to transform one sequence to another.

In such a case, the tier relationship changes to the index sequences of a lower tier subset differ by a lower monotonically decreasing function of a probability of sequence changes from each other than the index sequences of a

higher tier subset. Also, the truncated index sequences of a lower tier subset can differ by a lower monotonically decreasing function of a probability of sequence changes than the truncated index sequences of a higher tier subset.

The inventive set (and as it is selected in the inventive method) is preferably defined by a relationship of the distances between the index sequences of a subset, wherein the Sequence-Levenshtein distance between the index sequences of a higher tier subset is greater by at least 1, preferably 2, 3, 4, 5, 6, 7 or more, than the Sequence-Levenshtein distance between the index sequences of a lower tier subset.

When using other distances, one can also express that the Levenshtein distance between the index sequences of a higher tier subset is greater by at least 1, preferably 2, 3, 4, 5, 6, 7 or more, than the Levenshtein distance between the index sequences of a lower tier subset; or the Hamming distance between the index sequences of a higher tier subset is greater by at least 1, preferably 2, 3, 4, 5, 6, 7 or more, than the Hamming distance between the index sequences of a lower tier subset.

When using a sum or a maximum of probabilities (with values ranging from 0-1) then preferably the sum or a maximum of probability to transform one sequence index into another in a lower tier subset is greater by at least 0.00001, preferably at least 0.0001, or at least 0.001, or more, than the probability between the index sequences of a higher tier subset. This difference of the sum or a maximum of probabilities between the tiers may depend on the platform that is used and can be between 0.00001 and 0.9. If a logarithm to base "e" (natural logarithm) is used to that -log(P) is used to determine the difference in the distance between tiers then the value is preferably between 0.1 and 10.

For absolute distances within a tier, preferably the Sequence-Levenshtein distance between the index sequences of the highest tier subset is at least 4, such as 4, 5, 6, 7, 8 or more. The next lower tier would then in case of a difference between the tiers of 1 have a Sequence-Levenshtein distance between the index sequences of at least 3 and so on for the following tiers. The same applies for other integer distances (Levenshtein, Hamming). Preferably the lowest tier subset in the set has a Sequence-Levenshtein, Levenshtein or Hamming distance between its index sequences of at least 1, preferably, 2 or 3.

Since longer index sequences allow larger distances, it is preferred to provide a minimum length. Of course, shorter index sequences have also an advantage, i.e. lower costs as mentioned above. Thus, a compromise is selected. Preferably the index sequences have a length of at least 4, e.g., 4, 5, 6, 7, 8, 9, 10, 11, 12, 13, 14, 15, 16, nucleotides in contiguous sequence. Especially preferred is a length of at least 6, nucleotides length. The highest tier subset is also the smallest (fewest number of members). Each following lower tiered subset has more members but usually lower distances. In preferred embodiments, the highest tier subset comprises at least 2, e.g. 2, 3, 4, 5, 6, 7, 8, 9, 10, 11, 12 or more different index sequences. Preferably it comprises at least 4, different index sequences.

It is important that the subset structure is visible to a practitioner, i.e. that the index sequences are assigned to the subset to which they belong. For example, the oligonucleotides (with the index sequence) can be assigned to a subset by placement in a container that is labelled by a subset identifier. The identifier can be placed on the container or on a data carrier, like a manual, electronically or physically. The container can be a well in a well plate.

In further preferred embodiments, the sequences of the index sequences can be optimized for better stability or ability to be sequenced, for example. Common concepts are the optimization of a GC content and/or the avoidance of nucleotide repeats. Especially preferred is balancing of a distribution of all nucleotides of the genetic code across the different index sequences within a subset. Nucleotides of the genetic code are A, T or U, G, C. Usually one of T and U is used, with T being predominantly used, hence "T or U" is also written as "T(U)". Thus 4 different nucleotide types are usually found in the index sequence. T is found in DNA, U in RNA. The oligonucleotides can be DNA or RNA, for example, and/or comprise modified nucleotides, such as LNA.

Preferably the index sequences have a G/C content of 20% to 80% or 30% to 70 % or even 40% to 60%.

Preferably the index sequences do not contain repeats of the same nucleotide of at least 3 in length, i.e. no homopolymer triples.

Preferably the sequence GGC is avoided in some set-ups, especially for Illumina-based sequencing, since it is an Illumina-based error motif (ref. [3]).

Especially preferred, the index sequences of a subset have a balanced nucleotide distribution wherein the number of shared nucleotides at the same position within the index sequences between different index sequences is at most 0.5 times the number of index sequences in said subset. This criterion uses a sum (number of shared nucleotides per position) and accordingly compares it to a multiple (e.g. 0.5) of the number of index sequences in the subset (subset size) . The number of shared nucleotides at the same position, means that for each position, e.g. nucleotide (nt) 1, nt 2, nt 3, etc. the nucleotide type (A, T(U), G or C) is counted over all index sequences. Thus, when more index sequences are considered, the number increases. Hence, the criterion value (0.5 or lower, e.g. 0 to 0.5) is also multiplied by the number of index sequences that are considered. This is equivalent to using averages, which correspond to frequencies, that are compared to the value of 0.5 as preferred maximum frequency. This means that the number of shared nucleotides at the same position is then divided by the number of index sequences that are considered. This average is also referred to as nucleotide frequency (per position). Examples of such frequencies for each nucleotide are shown in Figures 8 to 11. Perfectly balanced nucleotides would mean that each nucleotide selected from A, T(U), G, C is equally distributed,

meaning a frequency of one quarter or 0.25, for all positions. Such optimal balancing is however not always possible since the sequence distance criterion needs also to be fulfilled. Hence dome deviations from perfect balancing are needed. This can be high for small subset sizes since a single index sequence deviating from the average can mean a larger difference from 0.25 (e.g. Fig. 8, showing a distribution in a subset of 4 index sequences). For larger subsets, it is usually possible to get closer to the desired 0.25 value. In preferred embodiments this criterion value or frequency is 0.4 or lower, e.g. in the range of 0.1 to 0.4, especially preferred for subset of size 8 or larger.

In addition or alternatively it is preferred when in at least 50% of the positions of the index sequences the nucleotide frequency for all index sequences of a subset for each nucleotide type is 0.5 or less, such as 0 to 0.5, preferably it is 0.4 or less, such as 0.1 to 0.4.

Particular preferred embodiments of the inventive sets comprise index sequences (or oligonucleotides comprising these index sequences) selected from any one of SEQ ID NO: 1 to 784, preferably of SEQ ID NO: 1 to 208. Preferably at least 10, preferably at least 15, at least 20, at least 30, at least 40, at least 50, at least 60, at least 70, at least 80 of SEQ ID NO: 1 to 784, preferably of SEQ ID NO: 1 to 208, are comprised in the set.

The present invention provides a computer-implemented method of generating a set of oligonucleotides comprising a plurality of subsets of oligonucleotides with a subset of index sequences. Everything disclosed for the set also applies to the method, e.g. the set with these parameters is obtained or the parameters are used and selected for in the method, such as the disclosed sequence distance determination methods.

The computer-implemented method comprises the steps of generating a first or higher tier subset of oligonucleotides with index sequences with a first or higher tier sequence distance to each other within the first or higher tier subset, wherein a sequence distance is a quantified amount of sequence changes that transforms one sequence into another or a monotonically decreasing function of a probability of sequence changes that transforms one sequence into another - as mentioned above -, generating a second or lower tier subset by including the first or higher tier subset and adding further oligonucleotides with index sequences with a second or lower tier sequence distance to each other within the second or lower tier subset, which second or lower tier sequence distance is a lower sequence distance than the first of higher tier sequence distance, whereby the second subset comprises oligonucleotides with index sequences that are not part of the first subset.

The terms higher tier subset and first subset can be used synonymously and refer to relative relationship between the subsets. Using the numerical values has the benefit of also referring to tiers that are further down than the second subset, such as a third subset that comprises the sequence indexes of the second subset (and hence also of the first) and additional sequence indexes. Consequently, its sequence distance requirement will likely be lower than the second tier's. This set-up corresponds to a set comprising at least 3 hierarchical tiers of subsets, which is a preferred embodiment. 3 hierarchical tiers in the higher-lower terminology means, that there is a first relationship between a higher ($1^{st}$) and a lower ($2^{nd}$) tier, as already stated and then another second relationship where this lower tier ($2^{nd}$ tier) becomes a higher tier for the next lower tier ($3^{rd}$ tier) .

The inventive set can have 2, 3, 4, 5, 6, 7, 8 or more hierarchical tiers, i.e. a first, second, third, fourth, fifth, sixth, seventh, eight or further tier wherein each tier subset in this order comprise the index sequences of the subset tier before and further index sequences as stated for the first and second tier (or higher and lower tier) respectively.

In preferred embodiments, the method comprises generating a lower tier subset by including a higher tier subset and adding further oligonucleotides with index sequences with a lower sequence distance than for the higher tier subset to each other within the lower tier subset. Likewise, the method may comprise generating a third subset by including the second subset and adding further oligonucleotides with index sequences with a third sequence distance to each other within the third subset, which third sequence distance is a lower sequence distance than the second sequence distance. This can apply to any further hierarchical tier of subsets as needed.

The step of generating the first, second (or further) subset of index sequences may comprise for one or more or each subset the step of selecting index sequences from a pool of different index sequence candidates. According to this embodiment, a pool of index sequences is generated as candidates for inclusion into subsets. These candidates usually have the desired length of the index sequences but lack the selected sequence distances in the pool of candidates. Said pool comprises several candidate index sequences in sufficient amount to fill a subset. Usually at least twice the number than the size of the subset is provided as pool to ensure that enough index sequence choices are available to provide the needed sequence distances and optionally other criteria as outlined herein in order for the subset. Preferably the pool of index sequences has at least by a factor of 2, more preferably 3, 4, 5, 6, 7, 8, 9, 10 or more, more members than the subset. The index sequences of the pool can be random or fulfil some other criteria, like a GC content of choice, omission of homopolymer triples, for example. The candidates are then added to the subset during its construction where the sequence distance criteria are adhered to (and other criteria, like balancing if so desired). If the criteria are not met, then other sequence index candidates are selected from the pool. If this does not suffice, a new sequence index candidates and/or new pools can be generated and used accordingly.

Preferably generating a first and/or second subset (or further analogously subsets) comprises selecting index sequences that comprise truncated index sequences and the truncated index sequences of at least one subset differ at

least by a non-zero number of sequence changes from each other truncated index sequence within said subset. The non-zero number of sequence changes can be a sequence distance of 1, 2, 3, 4, 5, 6, 7, 8 or more, especially preferred a Hamming, Levenshtein or Sequence-Levenshtein distance or a probability of changes as mentioned above. Preferably, the truncated index sequences of at least one subset differ by at least a number of sequence changes greater than 1 from each other truncated index sequence within said subset. The same as above with regard to truncated subsets applies. These allow the use of only a partial sequence (corresponding to the truncated sequence) in a method of assigning sequencing reads while a given sequence distance requirement between all truncated sequences of the subset are still met as described above.

In further preferred embodiments, correctable sequences are generated for an index sequence of a subset wherein said correctable sequences have a sequence distance that is less than half the sequence distance between the index sequences of said subset, and wherein the correctable sequences of different index sequences in said subset do not overlap. Such correctable sequences in the index sequences are also present in the set. Correctable sequences are sequences that can be associated to only one index sequence. This makes a sequence "correctable". A correctable sequence is thus a representation of an erroneously determined sequence that has one or more sequencing errors but when its sequence is correctable, it can still be assigned ("decoded") to one index sequence. In the method of generating index sequences for a subset, it can here be considered that around each index sequence a plurality of correctable sequence exists that still lead to one assignment when using the inventive set. This plurality is also referred to as a "decode sphere", using the analogy of a volume of sequences with a given distance to the index sequence in the centre of the sphere. In order to be assigned to one (and only one) index sequence, the distance should be less than half the sequence distance between the index sequences of said subset. This may not always be the case given the possibility of a violation of the triangle inequality mentioned above. Accordingly, the subset can take this possibility separately to the distance criterion between index sequences into account and maximize the number of correctable sequences or reducing the number of sequences that can be assigned to more than one, e.g. two or more, index sequences with equal distance (not correctable) . This is also referred to as decode sphere optimization, meaning that the overlap of two or more of such spheres is reduced or minimized. This can be done by selecting different index sequences to a given subset.

In a preferment generating a subset comprises selecting index sequences by adding an index sequence candidate and evaluating the sequence distance of the index distance candidate to all other pre-existing index sequences in the subset. The index sequence candidate is added to the index sequences of the subset if it fulfils a pre-set sequence distance requirement, such as any sequence distance property as discussed above. The index sequence candidate may be from the above-mentioned pool or not. Generally, this embodiment states that index sequence candidates are added step-wise during the build-up of a subset, wherein index sequences are added one after the other. An index sequence candidate is compared with the other pre-existing index sequences in the subset - if they exist (obviously this is not done for the first index sequence added to the subset). When the comparison results in a fulfilment of the distance requirement, and optionally other requirements, then the index sequence candidate is added to the subset. This process can be done for other subsets or even for subset candidates. A subset candidate is related as a subset but may not be included into the set if other subset candidates of the same size are also generated. Then usually a subset candidate is added to a set if it improves over another subset candidate. Improvement can be any criteria mentioned above, like improved balancing.

Such a balancing requirement that is preferably fulfilled is any one as mentioned above, preferably wherein an index sequence candidate contains for at least 50% of its positions a nucleotide type of the genetic code with the smallest frequency at the respective position in the pre-existing index sequences of the subset. This criterion is preferably applied to at least 25% of the index sequence candidates that are added last to the subset. As mentioned above, evaluating a frequency makes no sense when considering only one index sequence and has little value for small subsets under construction to which further index sequences or candidates are added. Balancing is best achieved when the subset is almost at its desired size, such as when it has 75% or more of its size, i.e. the remaining 25% are evaluated by this step. Especially preferred the last index sequence added to the subset is evaluated by this criterion.

In preferred embodiments an index sequence candidate is selected from a pool of index sequence candidates, wherein members of the pool of index sequence candidates fulfil a pre-set sequence distance requirement to each other member of the pool. Furthermore, an index sequence candidate of the pool is added to the index sequences of the subset, when the sum of the distances of the frequency of each nucleotide type of the genetic code to 0.25 at each position is the lowest for the index sequence candidate as compared to the other index sequence candidates of the pool. The distance of the frequency of each nucleotide type of the genetic code to 0.25 at each position can be measured as a sum of the absolute values of the difference, or preferably a squared or exponentiated difference, between the frequency of each nucleotide and 0.25 at each position, or as a probability distance measure between the frequency of each nucleotide and 0.25 at each position, where possible probability distance measures would be the Kullback-Leibler or Jensen-Shannon divergence. This absolute value of differences is a further preferred balancing option as discussed above. A frequency of 0.25 would be optimal balancing (when fulfilled for each position) but is seldom achievable. The closer the sequence index nucleotide frequencies are to 0.25, the better balances the subset.

A further preferred balancing criterion used in the method (and as found in the set) is wherein in at least 50% of the

positions of the index sequences the nucleotide frequency for all index sequences of a subset for each nucleotide type is 0.5 or less. Preferred embodiments of the balancing options are described above.

In preferred embodiments, the method of generating the inventive set comprises generating a plurality of subset candidates each with a given amount of members (index sequences). These competing subset candidates with the same size are compared to each other and one is selected for inclusion into the set, referred to as subset. Preferably the method comprises selecting a subset candidate as subset for the set, when said subset candidate has the lowest average over all index sequences per respective subset candidate of the sum of the absolute values of differences of the frequency of each nucleotide type of the genetic code at each position to 0.25. So, for each subset candidate the average absolute values of differences of the frequency of each nucleotide type of the genetic code to 0.25 for each position is summed for all its index sequences. The subset candidate that has a lower value (i.e. lower difference meaning better balanced - see above) is selected for inclusion into the subset. Preferably, the subset candidate with the lowest value is selected. If other criteria also are considered, it may be next to the lowest one even worse balancing. Preferably, the one subset candidate that is selected is among the better half (according to a lower value in this formula) of the considered subset candidates. The selection may be applied for complete subset candidates but it may also be apparent during build-up, e.g. when sequence index candidates are added successively as mentioned above, when during said build-up it becomes apparent that a given subset candidate will not result in a good value. Such worse performing subset candidates may be excluded from further consideration.

Alternatively or in combination, the method may comprise generating a plurality of subset candidates each with a given amount of members (index sequences) and selecting a subset candidate as subset for the set, wherein said subset candidate is selected by exclusion of other subset candidates, wherein a subset candidate is excluded when in a method that comprises adding index sequence candidates from a pool of index sequence candidates to the subset candidate, and optionally further adding comparative index sequences, the subset candidate has a higher average over all its index sequences sum of absolute values of differences of the frequency of each nucleotide type of the genetic code at each position to 0.25 as compared to another subset or subset candidate.

Such a selected subset is then added to the set. The "subset candidate has a higher average over all its index sequences sum of absolute values of differences of the frequency of each nucleotide type of the genetic code at each position to 0.25 as compared to another subset or subset candidate" is explained as above. The comparison to comparative index sequences means that when a subset candidate is generated by successive addition of sequence indexes and sequence index candidates, then this subset or subset candidate will only result in a full subset of its desired size when the last sequence index or sequence index candidate is added for consideration. To better evaluate intermediately added sequence index candidates, further comparative index sequences to fill the subset or subset candidate to its desired size can be added. The criteria, especially balancing criteria are then calculated for the sequence index candidate to each other sequence index and comparative sequence index. These comparative index sequences thus allow the simulation of a full subset to subset candidate without being used in the subset or subset candidate. They may of course be added to it, if they are selected as a sequence index candidate in a further step. The method may comprise removing subset candidates from further consideration at each step of consecutive build-up of the subset candidate if the balancing criterion is worse than that of other subset candidates or pre-existing subsets. Preferably, at least one subset candidate is excluded at each step of adding one sequence index to the subset candidate.

The set can be used for labelling a moiety, such as an oligonucleotide, a protein, a particle such as a nanoparticle, chemical compounds, especially a small-molecule compound of a size of 5 kDa or less etc.. Disclosed is a method of identifying the labelled moieties by determining the sequence of the index sequence that has been attached thereto and assigning the determined sequence to a known index sequence of the set. In particular, the invention provides a computer-implemented method of assigning sequencing reads (i.e., determined sequences) to a sample of oligonucleotides comprising the steps of

a) obtaining sample oligonucleotides from a plurality of samples,

b) selecting a subset of oligonucleotide index sequences from a set obtainable or obtained by the invention, wherein a subset is selected over another subset based on a higher sequence distance of the index sequences to each other within the selected subset; wherein a sequence distance is a quantified amount of sequence changes that transforms one sequence into another or a monotonically decreasing function of a probability of sequence changes that transforms one sequence into another, and wherein the selected subset has at least as many different index sequences as the number of samples of step a),

c) adding index sequences from said subset to each sample oligonucleotide thereof (which can be a fragment or fragmentation product), wherein the index sequences are indicative of the sample,

d) determining the sequence of the sample oligonucleotides or fragments of sample oligonucleotides and determining the index sequence,

e) assigning an obtained read sequence to a sample based on the determined index sequence or based on the index sequence which has the lowest sequence distance to the determined index sequence, wherein if two or more index

sequence have the same lowest distance then said obtained read is discarded; wherein optionally the sequence distance does not exceed a pre-set criterion value.

The method aims at retaining the sample association of oligonucleotides whose sequence is determined. The index sequences are thus labels that identify a sample association. This allows the concurrent sequence determination of many oligonucleotides from several samples in parallel (multiplex) since the sample association is maintained by the label information (to be determined index sequence). The method applies of course to any labelled moieties, not just oligonucleotides. Oligonucleotides read association is just the most common use for the inventive subsets.

It is not needed to use the entire set but only one of its subsets as long as the subset has the needed amount of index sequences (size). Of course, the entire set can be used, which represents in essence the lowest tier subset with the biggest size available in the set. In step a) the amount of samples to be labelled differently is ascertained. The reference to samples means of course samples that shall be distinguished in the method. In step b) a subset of the set is selected that can accommodate this amount of samples, i.e. the subset size is at least the amount of samples. To make best use of the inventive subset structure and in order to optimize the sequence distance between the index sequences of a subset, a subset is chosen over another subset based on a higher sequence distance of the index sequences to each other within the selected subset. Sequence distance is as defined and described above for the sets. This step means that - if possible, i.e. if subset size allows - a subset with higher sequence distances between its members is selected over another subset with a lower sequence distance between its members. In preferred embodiments step b) comprises selecting oligonucleotides with index sequences from a set obtainable or obtained according to the invention wherein a subset of oligonucleotides with the highest sequence distance of the index sequences within the subset is selected. I.e. the best subset with highest distance - as long as subset size allows - is selected. The selected subset shall have at least as many different index sequences as the number of samples of step a) that are needed to be identified or distinguished. The other subset may be used in other experiments or remain a surplus.

Step c) comprises adding index sequences from said subset to each sample oligonucleotide thereof. "Adding" means an attachment that connects the index sequences (as oligonucleotides) to the sample oligonucleotides or moieties so that this connection is maintained for assignment of the sequencing data. Usually a covalent attachment is used. In case of oligonucleotides, this may comprise a ligation. The sample oligonucleotide can be a fragment or fragmentation product of a once larger polynucleotide. Any sample preparation method is possible. For the sake of simplicity, the invention is only concerned with the preparation product that is going to be identified, such as in a sequencing step. This sequencing step can be a multiplex step as mentioned above where many oligonucleotides from different samples are pooled together and hence at this sept the labelling is needed. Any preparation of the sample moieties in steps where the samples are still kept separate do not require sample-specific labelling. E.g. an optional fragmentation without labels (index sequences) may be performed separate for each sample.

Step d) comprises determining the sequence of the sample oligonucleotides or fragments of sample oligonucleotides and determining the index sequence. These sequences (index sequence and sequence of the sample oligonucleotide) are usually determined in conjunction since usually they are after step c) on the same joined oligonucleotide molecule. The determined sequence that corresponds to the "sequence of the sample oligonucleotide" is also referred to as "read" or "sequencing read". Apart from sequencing errors or damages to the nucleotides during preparation, this determined sequence should correspond to the sequence of the sample oligonucleotide from the sample of step a).

Step e) comprises assigning an obtained/determined read sequence to a sample based on the determined index sequence or based on the index sequence which has the lowest sequence distance to the determined index sequence, wherein if two or more index sequences have the same lowest distance then said obtained read is discarded. The determined index sequence may fit perfectly error-free to an index sequence of a subset that is known. The advantage of the inventive set is that even in case of differences to errors, like sequencing errors or damages during preparation, a determined index sequence as obtained from step d) can be assigned to a known index sequence of the subset and hence a sample that it labels through "error-correction" as described above. I.e. due to large sequence distances between the index sequences of the subset during conception, and a large decode sphere, many different determined sequences can be assigned to the index sequence despite differences (i.e. in essence also distances to the index sequence) . This assignment usually selects the closest index sequence, i.e. the one with the smallest distance to the determined index sequence. If more than one index sequence shows the closest distance, i.e. an unambiguous assignment cannot be made, then the read may not be useable and can be discarded. Preferably this assignment of differing determined sequence has a cut-off value meaning that the sequence distance does not exceed a pre-set criterion value. If the distance exceeds such a cut-off, then the read may be discarded as well. Such a cut-off may be a distance of 3, 4, 5, 6, or 7 according to any distance measurement method as disclosed above, such as a Hamming distance, Levenshtein distance or a Sequence-Levenshtein distance.

Preferably the oligonucleotides that are sequenced comprise at least the index sequence, a sequence of the oligonucleotide of the sample, and optionally further an adapter sequence and optionally a universal identifier. An adapter may be a sequence that is used to hybridize primers to the oligonucleotide. It is usually the same sequence for all

oligonucleotides. A universal identifier may identify a sequencing experiment or multiplexing run and be specific for it but still be universal for all oligonucleotides that are sequenced together. Preferably, the oligonucleotide comprises at least two index sequences. This embodiment is also referred to as dual indexing (when two are used) or multiple indexing. Dual or multiple indexing allows further error identification or error correction, in particular, it allows the identification of errors due to index hopping (also referred to as "barcode hopping"), i.e. when one index sequence gets attached to an oligonucleotide of a wrong sample which it shall not label (see ref. [5], supplement) . When two or more index sequences are used, these are usually selected from different groups of index sequences, such as sets or subsets. It is common practice to label these groups as "i7" and "i5" or "left barcode" and "right barcode". For example, "i7" index sequences can be selected from SEQ ID NO: 1-104 and SEQ ID NO: 209-496, and "i5" index sequences can be selected from SEQ ID NO: 105-208 and SEQ ID NO: 497-784, or vice versa.

In further preferred embodiments determining a sequence of nucleotides of the index sequence comprises determining the sequence of the entire index sequence or a part thereof, wherein preferably a partial index sequence is determined in case a sequence distance of the partial index sequence to other partial index sequences within the same subset is larger than a non-zero criterion value. Parts of index sequences may have a sufficient distance to each other to allow assignment - of course of error-free determined sequence but in some case of error-corrected sequences as mentioned above as well. The partial index sequence is preferably a sequence of contiguous nucleotides of the index sequence. It may be 1, 2, 3, 4, 5, 6 or more nucleotides shorter than the index sequence. Preferably it still has a length of at least 4, 5, 6, 7, 8, 9, 10 or more nucleotides. Assignment with partial sequences works the same as for the full index sequences in that the partial sequence is compared to the corresponding part of the index sequence. In preferred embodiments the index sequences have truncated sequences that have a conceptional sequence distance between them as described above. As said, the truncated index sequences of the index sequences also have an adjusted sequence distance that is maintained for all truncated index sequences of a subset, meaning that it is possible to determine or consider during the use of the set only this partial sequence that corresponds to a truncated index sequence. Accordingly, in especially preferred embodiments, the partial index sequence has the sequence distance properties of a truncated index sequence as described above.

The present invention benefits also from the use of computers. Any method can be performed on a computer, especially the design of index sequences, of truncated index sequences, of subsets and the set and then its uses, such as the assignment of determined sequences to index sequences and subsets as described herein. Thus any method of the invention can be computer-implemented. The invention also provides a computer program product comprising instructions which, when the program is executed by a computer, cause the computer to carry out any method of the invention or their steps, in particular the ones named in this paragraph. The invention also provides a computer-readable storage medium comprising these instructions.

The following description of the invention uses detailed practical terminology. Of course, these descriptions and parts thereof can be combined with any of the general elements described above.

### 1. Nested barcode sets

A nested barcode set $B$ contains $S \geq 1$ nested subsets $B_1 \subset B_2 \subset \cdots \subset B_S$ such that the distance between barcodes within $B_i$ increases for smaller barcode sets. If the distance between barcodes $b, b'$ is given by $d(b, b')$ and $d_i = d(B_i) = \min_{b,b' \in B_i} d(b, b')$, then $d_1 > d_2 > ... > d_S$. A general outline on how such a nested barcode set can be created is given as follows. Let $n$ be the length of the barcodes and choose a sequence of distances, such that $d_1 > d_2 > ... > d_S$. We start by generating a barcode set $B_1$ with minimal inter-barcode distance equal to $d_1$. In some cases, this can be achieved by a lexicographical search [2]. If $d_1$ is chosen too large, it might not be possible to find a non-empty $B_1$ consisting of barcodes of length $n$. Then another $d_1$ shall be selected. However, in the following it is assumed that the sequence $d_1, ..., d_S$ has been labeled such that $d_1$ is the first distance for which a non-empty $B_1$ can be found. Since $d_1 > d_2$, barcode set $B_1$ can be used as a starting set in the search for $B_2$, which, again, can be used as a starting set in the search for $B_3$ etc. This process is visualized in Figure 2. Here, $B_1$ consists of 4 barcodes with label 1 with a minimal inter-barcode distance of $d_1$. $B_2$ is derived by using $B_1$ as a starting set and adding 4 barcodes with label 2. For $B_2$, one has $d_1 > d_2$. Finally, $B_3$ is derived by using $B_2$ as a starting set and adding 16 barcodes with label 3. This gives $d_1 > d_2 > d_3$ and $B_1 \subset B_2 \subset B_3$. The exact method by which $B_{i+1}$ is derived from $B_i$ depends on the barcode distance measure and the desired properties of the barcode sets $B_i$. To guarantee certain levels of cross-contamination, it might also be necessary to check further prefered properties of $B_i$ in addition to $d(B_i) = d_i$. These details will be discussed in Section 4.1.

### 2. Nested barcode sequences

Choosing a subset with appropriate size makes a nested barcode set adaptable to the number of samples in an experiment. In order to make the barcode set adaptable to the required level of cross-contamination we, further, designed our barcode sets such that the sequence of a barcode can be extended up to a specific length with a guaranteed increase in

the minimum distance between barcodes. The non-extended sequence corresponds then to a truncated or partal index sequence and the extended sequence to the index sequence. In addition, extended barcode sets retain the nested structure where the subsets in an extended barcode set consist of the extended barcodes in the subsets of the original barcode set. As for nested subsets, the process of barcode extension can be applied multiple times yielding nested barcode sequences. This means that more than one tier of truncation is possible. In case of several truncation steps, each truncated index sequence has a certain distance with each other truncated index sequence of the same tier within a subset. The overall structure of a nested barcode set with nested barcode sequences is visualized in Figure 3. The original barcode set, on the left side of the graphic, denoted by $B$ and with minimal inter-barcode distance $d(B)$ has a similar structure as set $B_3$ in Figure 2. Figure 3 shows that extending the sequences in $B$ by a sequence of nucleotides at the barcode ends, indicated by the arrow labeled "EXT" for extension, retains the nested subset structure in a new barcode set $EXT(B)$ with minmal barcode distance $d(EXT(B)) > d(B)$. In general, one has $EXT(B_1) \subset EXT(B_2) \subset \cdots \subset EXT(B)$ with $d(EXT(B_i)) > d_i$. A template for obtaining nested barcode sets with nested barcode sequences is given as follows. First, we choose the number of subsets $S$ and the number of subsequences $E$. The latter equals the number of extensions plus one. Next, we define the barcode lengths $n_j > 0$ for $j = 1, \ldots, E$. Since subsequent extensions increase the barcode length we require $n_{j+1} > n_j$. We, further, define the inter-barcode distances $d_{i,j} > 0$ for subsets $i = 1, \ldots, S$ and subsequences $j = 1, \ldots, E$. We require $d_{i,j} > d_{i+1,j}$, since increasing barcode set size decreases inter-barcode distance, and we require $d_{i,j+1} > d_{i,j}$, because extending barcode sequences increases inter-barcode distance. We search for a set of barcodes $C_1$ of length $n_E$ which satisfies $d(CUT^j(C_1)) = d_{1,E-j}$ for $j = 0, \ldots, E - 1$. Here $CUT$ is the opposite of $EXT$, i.e. $CUT^j$ removes the last $n_E - n_{E-j}$ nucleotides from sequences of length $n_E$ with $CUT^0 = id$, where id is the identity operator. Next, we search for a barcode set $C_2$ of length $n_E$ with $C_2 \supset C_1$ and $d(CUT^j(C_2)) = d_{2,E-j}$ for $j = 0 \ldots, E - 1$. We proceed in this manner until all $C_1 \subset C_2 \ldots \subset C_S$ have been found. This search is similar to the one discussed in Section 1 with the exception that we search for barcode sequences of length $n_E$ rather than $n$ which have to fulfill $d(CUT^j(C_i)) = d_{i,E-j}$ not just for $j = 0$ but for all $j = 0, \ldots, E - 1$. From $C_1 \subset C_2 \subset \cdots \subset C_S$ one can derive $B_i$ and $EXT^j$ by setting $B_i = CUT^{E-1}(C_i)$ and $EXT^j(B_i) = CUT^{E-1-j}(C_i)$ for $j = 0, \ldots, E - 1$. In the following, we will denote a nested set of barcodes with nested sequences as $EXT^j(B_i)$, where $j = 0, \ldots, E - 1$ and $i = 1, \ldots, S$.

### 3. Sequence distance measures

The inter-barcode distance $d(b,b')$ should reflect the frequency with which barcode $b$ changes into barcode $b'$. Since this is related to the similarity of the sequences of $b$ and $b'$, the sequence distance $d(b,b')$ is often chosen to be the minimal number of operations transforming sequence $b$ into sequence $b'$. The operations considered in such a sequence distance depend on the types of error expected during barcode processing. If only substitutions are expected, then $d(b,b')$ is the Hamming distance, see Section 3.1. If, additionally, insertions and deletions are taken into account then $d(b, b')$ is the Levenshtein or a related distance, see Sections 3.2 and 3.3. Since matches between sequences can also be counted in a sequence distance we will include matches as operations when we refer to error types or error classes, in the following.

In Section 3.4, $d(b,b')$ is the probability $p(b \rightarrow b')$ that $b$ transforms into $b'$. Contrary to a sequence distance, $p(b \rightarrow b')$ is not the minimal number of operations transforming $b$ into $b'$. Rather, it is the sum of the probabilities of all transformations that change $b$ into $b'$. Alternatively, one could also set $p(b \rightarrow b')$ to the average or maximum of the probabilities of all transformations changing $b$ into $b'$. The advantage of using $p(b \rightarrow b')$ as inter-barcode distance is that a high/small probability $p(b \rightarrow b')$ corresponds to a high/small transformation frequency. This is not always the case with a sequence distance, since barcodes with a high distance might change more frequently into each other than barcodes with a small distance, e.g. if error types have different probabilities.

### 3.1. Hamming distance

The Hamming distance between two sequences $b$ and $b'$ equals the number of substitutions that transform $b$ into $b'$. This is identical to the number of positions at which the sequences of $b$ and $b'$ differ. Barcodes $b$ = *AACGATAC* and $b'$ = *AAGGATTC,* for instance, differ at positions 3 and 7 and their Hamming distance is therefore 2. The Hamming distance is a distance in the proper mathematical sense, i.e. it is symmetric $d(b,b') = d(b',b)$, obeys the triangle-inequality $d(b,b') \leq d(b,c) + d(c,b')$, and equality of $b = b'$ is equivalent with $d(b,b') = 0$.

### 3.2. Levenshtein distance

The Levenshtein distance between $b$ and $b'$ equals the minimum number of substitutions, insertions and deletions needed to transform $b$ into $b'$. This number can be calculated with a dynamic programming algorithm as visualized in Figure 4. Here, $b_i$ and $b'_i$ stand for the i-th nucleotide of sequence $b$ and $b'$, which label the rows and columns of matrix $L$. There is an additional row and column labeled 0 before row $b_1$ and column $b'_1$. In the following, we will index the rows and columns of $L$ both by $0, b_1, b'_1, \ldots, b_n, b'_n$ as well as $0, \ldots, n$. Hence, we have $L(b_i,b'_j) = L(i, j)$. Initially, $L$ contains a single value $L(0,0) = 0$. The graph in the middle of Figure 4 shows that $L(i,j)$ is derived from $L(i,j - 1)$, $L(i - 1,j - 1)$ and $L(i - 1,j)$.

The transitions to $L(i,j)$ from $L(i,j-1)$ and $L(i-1,j)$ correspond to an insertion and deletion, respectively. The transition from $L(i-1,j-1)$ to $L(i,j)$ corresponds to a match if $b_i = b'_j$ and to a substitution otherwise. Matrix element $L(i,j)$ can be calculated as follows

$$L(i,j) = \min(L(i,j-1) + 1, L(i-1,j) + 1, L(i-1,j-1) + [b_i \neq b'_j]) \quad (1)$$

where [·] is the Iverson bracket, which equals 1 if the statement inside is true and 0 otherwise. Arguments of min in (1) with non-existing entries in $L$, i.e. for $i = 0$ and $j = 0$, are removed from the equation. The dynamic programming algorithm, given by (1) is performed row-wise, starting from the beginning of the row and running until the end. This is done for all rows in sequence starting at row 0. For row and column 0 this means that $L(0,i) = L(i,0) = i$. After the algorithm finished, the Levenshtein distance is contained in $L(n,n)$. Equation (1) shows that the penalties for insertions, deletions and substitutions are 1. These penalties can be modified if certain types of errors are more costly or frequent than others. If insertions and deletions have the same weight, the Levenshtein distance is symmetric otherwise it is non-symmetric. The other properties of a distance in the mathematical sense are always satisfied.

### 3.3. Fixed-frame Levenshtein distance

The ordinary Levenshtein distance is not ideal for measuring the inter-barcode distance since the sequencing frame for barcodes has a constant width. This means that if a barcode of length $n$ is expected, always $n$ nucleotides will be read by the sequencer. As a result, if a barcode has an insertion the last nucleotide of the barcode is shifted outside the barcode sequencing frame and is therefore not recorded. If the missing last nucleotide of the barcode is counted as a deletion error, as is the case for the Levenshtein distance, then every insertion which is not offset by a deletion would count as 2 errors. Similary, a deletion not offset by an insertion would count as 2 errors since the nucleotide which enters the frame at the end would be interpreted as an insertion. This artificial increase in inter-barcode distance could lead to the wrong conclusion that two barcodes are dissimilar and therefore unlikely to change into one another, when, in fact, they are similar and chances of barcode hopping are high. A more appropriate error distance is, therefore, a variant of the Levenshtein distance which takes into account that the size of the barcode sequencing frame is fixed. This distance, which has been variously referred to as FREE-divergence [3], Sequence-Levenshtein distance [1], or simply as modified Levenshtein distance [4, 6], can be derived by assigning a weight of 0 to insertions and deletions within the last row and column of matrix $L$ in Figure 4. Hence, insertions entering the sequencing frame after the end of the barcode are not counted as errors, just like deletions that occur due to the fact that the end of the sequencing frame has been reached. This fixed-frame Levenshtein (ff-Levenshtein) distance is not a proper metric as it does not satisfy the triangle inequality. This means that if two barcodes have distance 3 there might exist another barcode with distance 1 from both of them [3]. In this case, an inter-barcode distance of 3 does not guarantee that the barcode set can correct one error.

### 3.4. Sequence transition probability distance

Similarity of sequences, as measured by the minimal number of operations needed to transform sequences into each other, is not always directly correlated to the frequency with which sequences change into each other. Sequences with a high Levenshtein distance, for instance, might transform more frequently into each other than sequences with a small Levenshtein distance, if the operations that affect the transformation in the first case occur more often than the operations in the second case. Rather than generating barcode sets based on the minimal number of operations, it is therefore sensible to optimize barcodes with respect to the frequency or probability with which these operations occur. This approach will be pursued in this section by investigating inter-barcode distances based on sequence transition probabilities (STP).

In the following we will abbreviate matches, substitutions, insertions and deletions with $M,S,I$ and $D$. Given a probability distribution $p(o)$ with $o = M,S,D,I$, the probability that sequence $b$ changes into sequence $b'$, $p(b \to b')$, can be calculated by modifying the algorithm depicted in Figure 4. In this case, $L$ is initialized with $L(0,0) = 1$ and $L(i,j)$ is derived as follows

$$L(i,j) = L(i,j-1)\frac{p(I)}{4} + L(i-1,j)p(D) + L(i-1,j-1)([b_i \neq b'_j]\frac{p(S)}{3} + [b_i = b'_j]p(M)) \quad (2)$$

As before, terms on the right-hand side of (2) with an undefined value in $L$, i.e. for $i = 0$ and $j = 0$, are ignored. After algorithm completion, one has $p(b \to b') = L(n, n)$. In the following, $T$ will denote one of $M,S,I,D$ and the associated transition between elements of $L$. Thus, $T(i,j) = (i,j+1)$ and $T(i,j) = (i+1,j)$ for $T = I$ and $T = D$, whereas $T(i,j) = (i+1,j+1)$ for $T = M,S$. $T(i)$ and $T(j)$ will denote the first and second component of $T(i,j)$. In (2) $p(I)/4$ is the probability of inserting $b'_j$ after $b_i$ and $p(S)/3$ is

the probability of substituting $b_i$ with $b'_j$. In equation (2) therefore

$$p(b_{T(i)} \rightarrow b'_{T(j)}, T) = p(b_{T(i)} \rightarrow b'_{T(j)} | T, b_i) p(T | b_i) \quad (3)$$

where $p(T = o|b_i) = p(o)$ is independent of $b_i$, and $p(b_{T(i)} \rightarrow c|T,b_i)$ is uniform over all possible nucleotides $c$ given $T$ and $b_i$. For $T = I$, for instance, all 4 nucleotides can be inserted after $b_i$ and therefore $p(b_i \rightarrow c|I, b_i) = 1/4$. For $T = S$, on the other hand, $p(b_{i+1} \rightarrow c|S, b_i) = 1/3$ for $c \neq b_{i+1}$ and $p(b_{i+1} \rightarrow b_{i+1}|S, b_i) = 0$. If (3) does not hold or the factors on the right-hand side in (3) are not uniform then $p(b_i \rightarrow b'_{T(j)}, T)$ must be replaced by a more appropriate probability distribution. Similar to the fixed-frame Levenshtein distance, one can avoid penalization of insertions and deletions outside the sequencing frame by setting $p(I) = 1$ and $p(D) = 1$ in the last row and column of $L$. If barcodes are always followed by a certain sequence $a$, for instance an adapter sequence, then $a$ can be appended to $b$ to obtain a combined sequence $(b,a)$ for which the transition probability $p((b,a) \rightarrow b')$ can be calculated as before. One needs to note, however, that the matrix $L$ for the calculation of $p((b,a) \rightarrow b')$ is not square and that the final result is the element in the last row and column of $L$, i.e. $p((b,a) = b') = L(len(b) + len(a), len(b))$. Using the STP $p(b \rightarrow b')$, we define the distance between $b$ and $b'$ to be $d(b, b') = -\log_p(b \rightarrow b')$. This ensures that an increase in distance $d(b,b')$ always corresponds to a decrease in the probability that $b$ transforms into $b'$. In comparison to a sequence distance the values for $d(b,b')$ are not integers but real numbers greater than or equal to zero. The distance $d(b,b')$ is symmetric if $p(I)/4 = p(D)$. The equivalence $d(b, b') = 0 \Leftrightarrow b = b'$ will usually not be true, since $d(b, b) = 0$ only if $p(b \rightarrow b) = 1$, which requires $p(M) = 1$. In addition, the triangle inequality is not valid since $p(b_1 \rightarrow b_3) \geq p(b_1 \rightarrow b_2)p(b_2 \rightarrow b_3)$ does not hold in general.

**Estimating error class probabilities**

In this section the estimation of the error class probabilities $p(T = o|(i,j))$ for entries $(i,j)$ in matrix $L$ is described, given a set of sequences R derived by sequencing the barcodes in barcode set $B$. For this purpose, the probability of alignments of $b$ to $b'$ is shown. An alignment of $b$ to $b'$ is a path through matrix $L$ starting at (0,0) and ending at $(b_n, b'_n)$, such that an element $(b_i, b'_j)$ in the path is followed by $(b_{T(i)}, b_{T(j)})$ where $T = M,S,I,D$. In the following, $p(b \rightarrow b',(i,j))$ and $p(b \rightarrow b',!(i,j))$ will denote the probability that b transforms into b' with an alignment containing $(i,j)$ or not containing $(i,j)$, respectively. We will, further, denote by $p(b \rightarrow b',(i,j),T)$ the probability that $b$ transforms into $b'$ with an alignment containing $(i,j)$ followed by operation $T$. These probabilities will be calculated by making use of the following factorization.

$$p((b \rightarrow b', (i,j))) = p(b(1, \ldots, i) \rightarrow b'(1, \ldots, j)) p(b(i, \ldots, n) \rightarrow b'(j, \ldots, n) | S = (i,j)) \quad (4)$$

where $S = (i,j)$ signifies that the alignment of $b(i, \ldots, n) \rightarrow b'(j, \ldots, n)$ starts at $(i,j)$. To calculate $p(b(i, \ldots, n) \rightarrow b'(j, \ldots, n)|S = (i,j))$ we revert the algorithm for calculating $L$. For this purpose, the $n \times n$ matrix $L^{rev}_{b,b'}$ is initialized with $L^{rev}_{b,b'}(n,n) = 1$ and $L^{rev}_{b,b'}(i,j)$ is calculated as follows

$$L^{rev}(i,j) = L^{rev}(i,j+1)\frac{p(I)}{4} + L^{rev}(i+1,j)p(D)$$

$$+L^{rev}(i+1,j+1)([b_{i+1} \neq b'_{j+1}]\frac{p(S)}{3} + [b_{i+1} = b'_{j+1}]p(M)) \quad (5)$$

This algorithm proceeds row-wise from right to left and from last to first row. This procedure is shown in Figure 5. After algorithm completion, one has

$$p(b(i, \ldots, n) \rightarrow b'(j, \ldots, n) | S = (i,j)) = L^{rev}(i,j) \quad (6)$$

Since all paths in $L$ start at (0,0) it follows that $L(n,n) = L^{rev}(0,0)$. From (4) it follows that

$$p(b \rightarrow b', (i,j))) = L_{b,b'}(i,j) L^{rev}_{b,b'}(i,j) \quad (7)$$

and, further,

$$p(b \rightarrow b', (i,j), T) = L_{b,b'}(i,j) p(b_{T(i)} \rightarrow b'_{T(j)}, T) L^{rev}_{b,b'}(T(i,j)) \quad (8)$$

We use (7) and (8) to estimate the probability of *T* following (*i,j*). For this purpose, assume that *R* is a set of sequences derived by sequencing barcodes in barcode set *B*. We start with an initial estimate for $p^{(0)}(T = o|(i,j))$ and calculate iteratively

$$p^{(n+1)}(T = o|(i,j)) = \frac{\sum_{r,b} p^{(n)}(b \to r,(i,j),T=o|B)}{\sum_{o\prime,r,b} p^{(n)}(b \to r,(i,j),T=o\prime|B)} \quad (9)$$

We found that this procedure converges to the correct solution, as long as $p^{(0)}(T = o|(i,j))$ is not too far from the solution. Equation (9) calculates the probability of $T = o$ for each combination (*i,j*). In order to calculate the probability of $T = o$ after the *i*-th position of *b*, we used the following iterative scheme.

$$p^{(n+1)}(T = o|(i,\cdot)) = \frac{\sum_{r,b,j} p^{(n)}(b \to r,!(i,j-1),(i,j),T=o|B)}{\sum_{o\prime,r,b,j} p^{(n)}(b \to r,!(i,j-1),(i,j),T=o\prime|B)} \quad (10)$$

As in equation (9), we found that (10) converges to the correct solution if $p^{(0)}(T = o|(i,.))$ is not too far away. Finally, in order to calculate the overall probability of observing $T = o$ when aligning *b* with *b'* we used the following iterative procedure.

$$p^{(n+1)}(T = o) = \frac{\sum_{r,b,i,j} p^{(n)}(b \to r,(i,j),T=o|B)}{\sum_{o\prime,r,b,i,j} p^{(n)}(b \to r,(i,j),T=o\prime|B)} \quad (11)$$

As in (9) and (10), we found also that (11) converges to the correct solution. Since (10) and (11) accumulate data for multiple combinations of (*i,j*) these procedures need less data than (9) to converge. The procedure in (11) needs the least, as it accumulates data for all combinations of (*i,j*).

## 4. Barcode set generation

### 4.1. Minimizing cross-contamination

Cross-contamination occurs when the sequence $b_d$ read out for a barcode *b* coincides with the sequence of another barcode *b'*, or when a non-barcode sequence is read out which is corrected to the wrong barcode. The first type of cross-contamination, also called barcode hopping, is particularly problematic since it is not detectable. A barcode which transforms into another barcode appears to the user indistinguishable from the barcode into which it transformed. Barcode hopping can be reduced by searching for barcodes with a large distance from each other. In the case of a sequence distance, a large distance guarantees that barcodes are dissimilar and require a large number of errors to transform into each other. In the case of an STP distance a large inter-barcode distance is directly related to a low probability of barcode hopping. Typically, prior to generating a barcode set, the minimal inter-barcode distance (MIB) is specified. The generation then starts from an initial set of barcodes *B*, which can consist of a single random barcode or a predefined set of barcodes, see Section 4.2. The initial barcode set *B* is extended by adding a barcode *b'* with $d(b, b') \geq MIB$ and $d(b',b) \geq MIB$ for all $b \in B$. These two inequalities will be called the MIB condition. If the distance *d*(*b,b'*) is symmetric, only one of the inequalities in the MIB condition has to be verified. To find the next *b'* to add to *B,* elements in $B^c$, the complement of *B,* are examined in sequence. Here, the complement $B^c$ is the set of all sequences of the same length as elements in *B* which are not contained in *B.* The order in which elements in $B^c$ are examined can be random or follow a special ordering of *B*, such as lexicographical ordering [2], or be a combination of random and ordered. If all *b'* in $B^c$ are examined, the sequence in which the *b'* are processed is irrelevant. If *b'* satisfies the MIB condition, it is added to the set of potential barcodes *P*. Once *P* satisfies the required properties, e.g. that it is not empty, or that it cannot be further extended in size, then $b' \in P$ is selected by another, possibly random, procedure and added to *B*. At this stage, *B* might already fulfill all the requirements that one has for a barcode set, e.g. sufficient size, in which case the search would terminate. This barcode search can be summarized as follows.

### Search algorithm 1

1. Specify barcode length *n* and *MIB* > 0.
2. Initialize barcode set *B* with a random barcode *b* or with a predefined set of barcodes.

3. Initialize $P$, the set of potential barcodes, with $P = \emptyset$.
4. Examine sequences $b' \in B^c$, where $B^c$ is the complement of $B$ in the set of sequences of length $n$, i.e. the set of all sequences of length $n$ not contained in $B$. If $d(b, b') \geq MIB$ and $d(b',b) \geq MIB$ for all $b \in B$, add $b'$ to the set of potential barcodes $P$. Repeat this step until $P$ fulfills the required properties or all elements in $B^c$ have been examined.
5. If $P$ does not fulfill the required properties terminate.
6. Select $b' \in P$ and add to $B$. If $B$ fulfills the required properties terminate, otherwise goto step 3.

If we search for nested barcode sets with nested sequences the above procedure has to be modified. Here, we will use the notation from Section 2., i.e. $S$ is the number of subsets and $E$ is the number of subsequences. In the previous algorithm, barcode length $n$ is replaced by barcode lengths $n_1, \ldots, n_E$ and minimal inter-barcode distance MIB is replaced by inter-barcode distances $d_{i,j}$, where $i = 1, \ldots, S$ and $j = 1, \ldots, E$. Further, the MIB condition has to hold for all $EXT^{j-1}(B_i)$ and $d_{i,j}$. As noted in Section 2, the search for nested barcode sets proceeds by searching for sets $C_1 \subset \cdots C_S$ of barcodes with length $n_E$. In particular, a sequence $c'$ is considered a potential barcode candidate for $C_i$ only if $d(CUT^j(c),CUT^j(c')) \geq d_{i,E-j}$ and $d(CUT^j(c'),CUT^j(c)) \geq d_{i,E-j}$ for all $c \in C_i$ and $j = 0, \ldots, E - 1$. The modified version of the above barcode search for nested barcode sets with nested sequences now looks as follows.

**Search algorithm 2**

1. Specify number of subsets $S \geq 1$, number of subsequences $E \geq 1$ and for $i = 1, \ldots, S$ and $j = 1, \ldots, E$ barcode lengths $n_j > 0$ with $n_{j+1} > n_j$, and inter-barcode distances $d_{i,j} \geq 0$ with $d_{i,j} > d_{i+1,j}$ and $d_{i,j+1} > d_{i,j}$.
2. Set $i = 1$ and initialize barcode set $C$ with a random barcode $c$ of length $n_E$ or with a predefined set of barcodes of length $n_E$.
3. Initialize $P$, the set of potential barcodes, with $P = \emptyset$.
4. Examine sequences $c' \in C^c$, where $C^c$ is the complement of $C$ in the set of sequences of length $n_E$. If $d(CUT^j(c),CUT^j(c')) \geq d_{i,E-j}$ and $d(CUT^j(c'),CUT^j(c)) \geq d_{i,E-j}$ for $j = 0, \ldots, E - 1$ and all $c \in C$ add $c'$ to the set of potential barcodes $P$. Repeat this step until $P$ fulfills the required conditions or all elements in $C^c$ have been examined.
5. If $P$ does not fulfill the required conditions goto step 7.
6. Select $c' \in P$ and add $c'$ to $C$. If $C$ fulfills the requirements for set $C_i$ goto step 7. Otherwise, goto step 3.
7. Assign $C_i = C$ and: if $i < S$ set $i = i + 1$ and goto step 3, if $i = S$ set $B_i = CUT^{E-1}(C_i)$ and $EXT^j(B_i) = CUT^{E-1-j}(C_i)$ for $j = 0, \ldots, E - 1$, then terminate.

It should be noted that the barcode sets produced by Search algorithm 2 contain, for $S = 1$ and $E = 1$, the barcode sets produced by Search algorithm 1. For $E = 1$ and $S > 1$, Search algorithm 2 produces nested barcode sets without nested sequences and for $S = 1$, $E > 1$ Search algorithm 2 produces nested sequences without nested subsets. Hence, Search algorithm 2 can be used to selectively generate barcode sets with multiple nested subsets and/or sequences.

In the following, we will refer to a nested barcode set with $S$ subsets, $E$ subsequences with lengths $n_1, \ldots, n_E$, where $d(b,b')$ is a distance of type DTYPE, as DTYPE-S ($n_1, \ldots, n_E$). Hence, if $EXT^j(B_i)$ has been designed for an ff-Levenshtein distance $d(b,b')$, with $S = 5$ and $n_1 = 8$, $n_2 = 10$ and $n_3 = 12$ the nested barcode set will be referred to as ff-Levenshtein-5(8,10,12). If $E = 1$, we will use DNAME($n_1$), rather than DNAME-1($n_1$), to refer to a set of barcodes with length $n_1$. Hence, Hamming(6) refers to a set of barcodes of length 6 which has been designed for the Hamming distance.

The second type of cross-contamination, mentioned at the beginning of this section, is the result of false error-correction. Here, the sequence read out (determined sequence) for a barcode is incorrect but does not coincide with another barcode. The error occurs when the non-barcode sequence is assigned to the wrong barcode by an error-correction procedure. The set of sequences which are corrected to a barcode $b$ is the decode sphere of the barcode. Hence, to guarantee the proper correction of at least a minimal number of errors (MEC), one has to check that sequences generated with up to MEC errors from a barcode $b$ lie in the decode sphere of $b$ and that decode spheres for different barcodes do not overlap. Non-barcode sequences $c$ are usually corrected to the barcode with minimal distance. If distance $d$ obeys the triangle inequality, $d(b,b') \geq MIB$ and $d(b,c) < MIB/2$ imply that $d(c,b') > MIB/2$. Hence, if $d$ is symmetric and $c$ has been generated from b with at most $MEC < MIB/2$ errors then $c$ lies only in the decode sphere of $b$. If distance $d$ is not symmetric, as in the case of a Levenshtein distance with non-equal weights for insertions and deletions, one has to check, additionally, whether $d(c,b) < MIB/2$. This implies $d(b',c) > MIB/2$ and, therefore that $c$ lies only in the decode sphere of b. For a distance that does not obey the triangle inequality, if is usually not sufficient to ensure that $c$ has been generated from $b$ with at most $MEC < MIB/2$ errors. For the fixed-frame Levenshtein distance, for instance, one can find sequences $b,b'$ and $c$ such that $d(b,b') = 3$, but $d(b,c) = 1$ and $d(b',c) = 1$, see [3]. In this case, therefore, with $MIB = 3$, $MEC = 1$ and $MEC < MIB/2$ the decode spheres overlap. Hence, if the triangle inequality does not hold, it is usually necessary to verify directly that decode spheres do not overlap. In the following, we will always assume that a non-barcode sequence $c$ is corrected to the $b$

$\in B$ with the smallest distance. We will, further, denote by $D(b,r)$ the sphere of radius $r$ around $b$, which is the set $D(b,r) = \{b':len(b') = n, d(b,b') \leq r\}$, and write $D(B, r) = \cup_{b\in B} D(b,r)$ for the sphere of radius $r$ around barcode set $B$. The search for barcode sets in [3] proceeds by looking for $b'$ with $d(b, b') \geq MIB$ and $d(b',b) \geq MIB$, such that $D(B, MEC) \cap D(b',MEC) = \emptyset$. This procedure can be summarized as follows.

**Search algorithm 3**

1. Specify barcode length $n$, $MIB \geq 0$ and $MEC \geq 0$ with $MEC < MIB$.
2. Initialize barcode set $B$ with a random barcode $b$ or with a predefined set of barcodes. If decode sphere $D(B,MEC)$ of $B$ is unknown calculate decode sphere $D(B,MEC)$.
3. Initialize $P$, the set of potential barcodes, with $P = \emptyset$.
4. Examine sequences $b' \in B^c$, where $B^c$ is the complement of $B$ in the set of sequences of length $n$. If $d(b, b') \geq MIB$ and $d(b',b) \geq MIB$ for all $b \in B$ and $D(B,MEC) \cap D(b',MEC) = \emptyset$ add $b'$ to the set of potential barcodes $P$. Repeat this step until $P$ fulfills the required properties or all elements in $B^c$ have been examined.
5. If $P$ does not fulfill the required properties terminate.
6. Select $b' \in P$ and add to $B$. If $B$ fulfills the required properties terminate, otherwise goto step 3.

This barcode search is computationally more expensive than Search Algorithm 1. This is because the calculation of $D(B,MEC) \cap D(b',MEC)$ requires the calculation of the distance of $b'$ to all sequences in $D(B,MEC)$. For nested barcode sets with nested sequences the algorithm above has to be adapted as follows. First, one needs to define distances $f_{i,j}$ such that $f_{i,j} < d_{i,j}$ for $i = 1, \ldots, S$ and $j = 1, \ldots, E$. For these distances, we require that error correction maps b' a sequence of length $n_j$ to $EXT^j(b)$, if $D(EXT^j(b),b') < f_{i,j}$ and $b \in B_i$. More generally, we require that $D(EXT^j(b),f_{i,j}) \cap D(EXT^j(B\backslash b), f_{i,j}) = \emptyset$. The complete algorithm is given as follows.

**Search algorithm 4**

1. Specify number of subsets $S \geq 1$, number of subsequences $E \geq 1$ and for $i = 1, \ldots, S$ and $j = 1, \ldots, E$ barcode lengths $n_j > 0$ with $n_{j+1} > n_j$, and inter-barcode distances $d_{i,j} \geq 0$ with $d_{i,j} > d_{i+1,j}$ and $d_{i,j+1} > d_{i,j}$. Specify, further, error-correction distances $f_{i,j} \geq 0$ with $f_{i,j} < d_{i,j}$.
2. Set $i = 1$ and initialize barcode set C with a random barcode $c$ of length $n_E$ or with a predefined set of barcodes of length $n_E$. Calculate decode spheres $D(CUT^j(C),f_{i,E-j})$ for $j = 0, \ldots, E - 1$.
3. Initialize $P$, the set of potential barcodes, with $P = \emptyset$.
4. Examine sequences $c' \in C^c$, where $C^c$ is the complement of $C$ in the set of sequences of length $n_E$. If $d(CUT^j(c), CUT^j(c')) \geq d_{i,E-j}$ and $d(CUT^j(c'),CUT^j(c)) \geq d_{i,E-j}$ for $j = 0, \ldots, E - 1$ and all $c \in C$, and if, further, $D(CUT^j(C),f_{i,E-j}) \cap D(CUT^j(c'), f_{i,E-j}) = \emptyset$ for $j = 0, \ldots, E - 1$ add $c'$ to the set of potential barcodes $P$. Repeat this step until $P$ fulfills the required conditions or all elements in $C^c$ have been examined.
5. If $P$ does not fulfill the required conditions goto step 7.
6. Select $c' \in P$ and add $c'$ to $C$. If $C$ fulfills the requirements for set $C_i$ goto step 7. Otherwise, goto step 3.
7. Assign $C_i = C$ and: if $i < S$ set $i = i+1$ and goto step 3, if $i = S$ set $B_i = CUT^E(C_i)$ and $EXT^j(B_i) = CUT^{E-j}(C_i)$ for $j = 0, \ldots, E - 1$, then terminate.

Similar to Search algorithm 2, also Search algorithm 4 can be used to selectively generate barcode sets with multiple nested subsets and/or sequences by appropriately choosing parameters $S$ and $E$.

Nested sets of barcodes $EXT^j(B_i)$ with $d_{i,j} > d_{i+1,j}$, $d_{i,j+1} > d_{i,j}$ and $f_{i,j} \leq d_{i,j}$ can also be generated without specifying the $d_{i,j}$ and $f_{i,j}$ in step 1 in Search algorithms 2 and 4. The corresponding search algorithm is given below, where we will use the notation $CUT^j(c, c') = (CUT^j(c),CUT^j(c'))$ and $D_c(c') = \min_{c\in C}\min\{d(c,c'),d(c',c)\}$. The latter can be interpreted as the distance of $c'$ to $C$.

**Search algorithm 5**

1. Specify number of subsets $S > 2$, number of extensions $E \geq 0$ and for $i = 1, \ldots, S$ and $j = 1, \ldots, E + 1$ barcode lengths $n_j > 0$ with $n_{j+1} > n_j$.

2. Set $i = 1$ and initialize barcode set C with a random barcode $c$ of length $n_{E+1}$. Initialize $X$, the set of barcodes to be excluded from the search, with $X = \emptyset$.
3. Assign $c_i = \mathrm{argmax}_{c' \in C^c \wedge c' \notin X} D_C(c')$.
4. If $i = 1$ goto step 6.
5. Set $d_{i,j} = \min_{c' \in C}\{d(CUT^{E+1-j}(c_i,c')),\ d(CUT^{E+1-j}(c',c_i))\}$. If $d_{i,j} < d_{i-1,j}$ and $d_{i,j} < d_{i,j+1}$ for $j = 1, \ldots, E + 1$ goto step 6, otherwise set $X = X \cup c_i$ and goto step 3 unless $X = C^c$.
6. Set $C = C \cup c_i$, $C_i = C$, $d_{i,j} = \min_{c' \in C}\{d(CUT^{E+1-j}(c_i,c')),d(CUT^{E+1-j}(c_i,c_i))\}$ and $f_{i,j} = \max\{f > 0: D(CUT^{E+1-j}(C_{i-1}),f) \cap D(CUT^{E+1-j}(c_i),f) = \emptyset\}$. If $i < S$ set $i = i + 1$, $X = \emptyset$ and goto step 3, otherwise terminate.

The above procedure generates a sequence of nested barcode sets $EXT^j(B_i)$ with $|B_i| = i + 1$. The maximum in step 3 of the above algorithm might not be unique. In this case, $c_i$ has to be selected from the set of all $c'$ with maximal distance from $C$. This happens, in particular, when distance $d(c,c')$, such as a sequence distance, takes on a finite number of values. If values of $d(c, c')$ are continuous, as for an STP distance, the maximum in step 3, and therefore $c_i$, will generally be unique.

### 4.2. Avoiding unwanted barcode sequences

There are various situations in which one might want to remove sequences from the set of possible barcodes. This is, for instance, the case if a sequence has low amplification efficiency. Barcoding a sample with such a sequence can result in the sample obtaining considerably less lane share than other multiplexed samples. To avoid such a sequence, the barcode search in Section 4.1 has to be slightly modified. Instead of examining all sequences $b'$, or $c'$ in $B^c$ or $C^c$, respectively, one should examine only $b'$ or $c'$ other than the sequences to be excluded. Another situation causing problems in demultiplexing occurs when sequencing produces incorrect but frequent sequences which are not associated with any barcode. Such sequences can, for instance, appear when the index is not properly ligated to the fragment. In this case, some sequencers will often produce a sequence consisting almost entirely of G's. Such artificial sequences, not associated with a barcode, will have a negative impact on error-correction if they are assigned to the closest barcode. In order to avoid this problem, such artificial sequences should not be contained in the decode sphere of any barcode. If artificial sequences can themselves have variants, the sphere around the artificial sequences containing these variants should not overlap with the decode sphere of any barcode. This problem can be addressed by adding the artificial sequences (comparative index sequences) to the initial set of barcodes from which the search in Section 4 generates a full barcode sets. This guarantees that the decode spheres of the resulting barcodes do not overlap with the decode spheres of the artificial sequences. Upon completion of the barcode search, the artificial sequences are removed from the final barcode set. As artificial sequences, it is possible to add sequences consisting entirely of either A, C, G or T to barcode set $B_1$ prior to starting the barcode search.

### 4.3. Positional nucleotide balancing

The barcode set used to multiplex the samples in an RNA-Seq run should have a balanced nucleotide distribution at each barcode position. An uneven distribution can lead to low quality scores or a low pass filter rate. To achieve a balanced nucleotide distribution for the $s$ samples in an RNA-Seq run with a nested barcode set $EXT^j(B'_k)$ one has to select an appropriate barcode set $B$ with $|B| = s$ from $B'_k$ where $|B'_{k-1}| < s \leq |B'_k|$. To obtain such a set $B$, the following selection method can be used. It evaluates all possible subsets $B \subset B'_k$ satisfying $|B| = s$ with the help of an A* search. For commonly used sample numbers $s_i < s_{i+1}$ multiplexed in an RNA-Seq run, we used this selection method to obtain a nested barcode set $EXT^j(B_i)$ with $|B_i| = s_i$. We start by selecting $B_1$ from the $B'_k$, where $|B'_{k-1}| < s_1 \leq |B'_k|$. Subsequently, we select $B_{i+1}$ from $B'_k$, where $|B'_{k-1}| < s_1 \leq |B'_k|$ such that $B_i \subset B_{i+1}$. Since multiple $B_i$ can satisfy $B'_{k-1} \subset B_i \subset B'_k$ it is possible that $d_i = d_{i+1}$ for some of the $i$. Such a nested barcode set is, therefore, a slight variation of the concept considered so far.

The nucleotide distribution of a barcode set $B$ at position $p$ is given by $\delta(B,p,n) = \Sigma_{b \in B}[b(p) = n]/|B|$, where $n = A, C, G, T$. If $p,n$ are not relevant this distribution will simply be written as $\delta(B)$. We measure the distance $D(\delta_1,\delta_2)$ between two positional nucleotide distributions $\delta_1(p,n)$ and $\delta_2(p,n)$ as follows $D(\delta_1,\delta_2) = \Sigma_{p,n}(\delta_1(p,n) - \delta_2(p,n))^2$, and denote by $D(\delta)$ the distance of $\delta$ to the uniform positional nucleotide distribution (UPND), i.e. $D(\delta) = \Sigma_{p,n}(\delta(p,n) - 1/4)^2$. To find a barcode set $B$ of size $s$ within another barcode set $B'$ we employ the following A* search. Assume that a subset $\beta \subset B'$ with $|\beta| < s$ has already been selected. We want to find a lower bound for $D(\delta(B))$, given that $\beta \subset B$. The barcode sequence $b$ producing the smallest value of $D(\delta(\beta \cup b))$ contains at each position $p$ the nucleotide with the smallest frequency at $p$ in $\beta$, i.e. $b(p) = \mathrm{argmin}_n \delta(\beta,p,n)$. If $n$ is not unique, it is chosen randomly from all $n$ minimizing $\delta(\beta,p,n)$. The nucleotide sequence $b$ is not necessarily contained in $B'$. Hence, adding a single barcode from $B'$ to $\beta$ will potentially yield a distance from the UPND which is larger than $D(\delta(\beta \cup b))$. If we set $b_1 = b$, $\beta_1 = \beta$ and $\beta_{i+1} = \beta_i \cup b_i$, repeated application of this construction produces a sequence of barcode sequences $b_1, \ldots, b_{|B|-|\beta|}$. We use $D(\delta(\beta \cup \bigcup_{i=1}^{|B|-|\beta|} b_i)$ as the lower bound for the distance

from the UPND for a subset $B \subset B'$ with $\beta \subset B$. In our A* search we use a depth-first approach. To find the $B \subset B'$ of size $s$ with minimal distance $D(\delta(B'))$ we sequentially generate all subsets of barcodes of size $s$. Subsets of size $s$ are themselves generated by adding one barcode after another. When a new barcode $b$ is added to a subset $\beta$, we calculate the lower bound above for $D(\delta(B))$ given that $\beta \cup b \subset B$. If this estimate lies above or is equal to the distance for a set of size $s$ for which the distance to the UPND has already been calculated then $\beta \cup b$ and all subsets containing $\beta \cup b$ are removed from the search. This considerably reduces the number of barcode sets which have to be examined and allows, in many cases, to find a barcode set $B$ with $|B| = s$ and minimal $D(\delta(B))$.

**5. Barcode arrangement on well-plates**

As mentioned in the introduction, the barcode sets $B_1 \subset B_2 \subset \cdots$ are used for experiments with different numbers of samples. If a user has at most $|B_1|$ samples they will use barcodes from set $B_1$. If they have more than $|B_1|$ and at most $|B_2|$ samples they will use barcodes from set $B_2$. The minimal number of barcodes which can, at least in theory, have a UPND is 4. Hence, $B_1$ should contain at least 4 barcodes. Since the number of samples in an experiment is often a multiple of 8, it is sensible to require that $B_i = m_i 8$ for $i > 1$, where $m_i$ is a positive integer. To make pipetting such barcode sets more convenient or easier to automate, it is, further, sensible to arrange them on well-plates such that barcodes in $B_i$ are grouped together. A possible arrangement on an 8x12 well-plate, where barcode sets with sizes 4, 8, 16, 24, 96 are grouped in columns is shown in Figure 6. Here, wells A1-D1 contain barcodes in $B_1$, wells A1-H1 contain barcodes in $B_2$, columns 1 and 2 contain barcodes in $B_3$ and columns 1, 2 and 3 contain barcodes in $B_4$. The complete set of barcodes in all wells is $B_5$. If the barcode sets can be extended to $C_1 \subset C_2 \subset \cdots$, then the barcodes of length $n + me$ in the $C_i$ are contained in the wells of the well-plate and grouped as described for the $B_i$.

**6. Reducing and quantifying cross-contamination with dual indices**

Barcodes can be used as single or dual indices on an Illumina sequencer [5]. In a single index RNA-Seq run a barcode $b_{i7}$ from barcode set $B_{i7}$ (e.g. selected from SEQ ID NO: 1-104 and SEQ ID NO: 209-496), "i7 index", before the P7 adapter. In a dual-index run a second barcode $b_{i5}$ from another barcode set $B_{i5}$ (e.g. selected from SEQ ID NO: 105-208 and SEQ ID NO: 497-784), "i5 index", before the P5 adapter. This setup is visualized in Figure 7. Here, barcode sequences $b_{i7}$, $b_{i5}$ and barcode sets $B_{i5}$, $B_{i7}$ might be identical. Figure 7 shows that $b_{i7}$ and $b_{i5}$ are sequenced in the same direction as read 1 and read 2 producing sequences $b_{i7,d}$ and $b_{i5,d}$, where, potentially, $b_{i7} \neq b_{i7,d}$ and $b_{i5} \neq b_{i5,d}$. The proportion of $(b_{i7,d}, b_{i5,d}) \in B_{i7} \times B_{i5}$ will be called the purity of an RNA-Seq run. To multiplex samples in a dual-index RNA-Seq run, a subset of barcode tuples $C \subset B_{i7} \times B_{i5}$ of the same size as the number of samples has to be selected. Each sample in the RNA-Seq run is then labeled with its unique barcode combination $(b_{i7}, b_{i5}) \in C$. To minimize cross-contamination, $C$ should be chosen such that $(b_{i7}, b_{i5}) \in C$ and $(b_{i7}', b_{i5}') \in C$ implies $b_{i7} \neq b_{i7}'$ and $b_{i5} \neq b_{i5}'$. Such a $C$ will be called unique component dual index barcode set (UCDI). A UCDI $C$ ensures that barcode hopping in either $b_{i7}$ or $b_{i5}$ will produce a barcode tuple not contained in $C$. For a UCDI $C$, therefore, hopping of a single barcode is detectable. This is an advantage over single-index RNA-Seq runs, where barcode hopping is not detectable. A UCDI, however, cannot correct hopping of a single barcode, since for $(b_{i7,d}, b_{i5,d}) \not\in C$ and $(b_{i7,d}, b_{i5,d}) \in B_{i7} \times B_{i5}$ it is, in general, impossible to say whether $b_{i7,d} \neq b_{i7}$ or $b_{i5,d} \neq b_{i5}$. As a consequence, fragments with associated $(b_{i7,d}, b_{i5,d}) \notin C$ and $(b_{i7,d}, b_{i5,d}) \in B_{i7} \times B_{i5}$ have to be removed from further downstream analysis. A tuple $(b_{i7,d}, b_{i5,d}) \notin C$ is obtained in any of the three mutually exclusive cases,

1.

$$b_{i7,d} \neq b_{i7} \land b_{i5,d} = b_{i5}$$

2.

$$b_{i5,d} \neq b_{i5} \land b_{i7,d} = b_{i7}$$

3.

$$b_{i7,d} \neq b_{i7} \land b_{i5,d} \neq b_{i5} \land (b_{i7,d}, b_{i5,d}) \not\in C$$

Hence, the probability of observing $(b_{i7,d}, b_{i5,d}) \notin C$ is the sum of the probabilities of the cases above. In a reasonably clean RNA-Seq run the simultaneous hopping of two barcodes is highly unlikely and the probability that $b_{i7,d} \neq b_{i7} \land b_{i5,d} \neq b_{i5}$ is therefore negligible. Similarly, $b_{i7,d} \neq b_{i7}$ implies $b_{i5,d} = b_{i5}$ and $b_{i5,d} \neq b_{i5}$ implies $b_{i7,d} = b_{i7}$. Hence, for a

reasonably clean RNA-Seq run the probability that $(b_{i7,d}, b_{i5,d}) \notin C$ is approximately given by the following sum

$$p((b_{i7,d}, b_{i5,d}) \not\in C) = p(b_{i7} \neq b_{i7,d}) + p(b_{i5} \neq b_{i5,d}) \qquad (12)$$

Hence, an upper threshold for the probability of barcode hopping in $B_{i7}$ and $B_{i5}$ is given by

$$p(b_{i7/5} \neq b_{i7/5,d}) \leq p((b_{i7,d}, b_{i5,d}) \not\in C) \qquad (13)$$

Here *i7/5* means that *i7* and *i5* can be used interchangeably. The upper bound in (13) is tight only if one of $p(b_{i7/5} \neq b_{i7/5,d})$ equals zero. This means that only one barcode set $B_{i7/5}$ is responsible for all the errors. In practice, however, it is more likely that both barcode sets contribute in equal measure to the observed errors and the probabilities on the left-hand side of (13) will therefore be closer to half the right-hand side of (13). For $(b_{i7,d}, b_{i5,d}) \in B_{i7} \times B_{i5}$ equation (13) changes into

$$p(b_{i7/5} \neq b_{i7/5,d}, (b_{i7,d}, b_{i5,d}) \in B_{i7} \times B_{i5}) \leq p((b_{i7,d}, b_{i5,d}) \not\in C, (b_{i7,d}, b_{i5,d}) \in B_{i7} \times B_{i5}) \quad (14)$$

The left-hand side of (14) is the probability that barcode hopping takes place in $b_{i7}$ or $b_{i5}$, while the right-hand side of (14) can be estimated from demultiplexing an RNA-seq run with respect to all barcode combinations in $B_{i7} \times B_{i5}$ and calculating the ratio of the $(b_{i7,d}, b_{i5,d}) \notin C$ in the tuples with $(b_{i7,d}, b_{i5,d}) \in B_{i7} \times B_{i5}$. Hence, a dual-index RNA-Seq run can be used to derive an upper bound for the probability of barcode hopping in a single-index experiment. The probability to observe barcode hopping in a dual-index RNA-Seq run using barcodes from $C \subset B_{i7} \times B_{i5}$ is the product of probabilities $p(b_{i7/5} \# b_{i7/5,d})$ satisfying (12) . This product is maximized if $p(b_{i7/5} \neq b_{i7/5,d}) = p((b_{i7,d}, b_{i5,d}) \notin C)/2$. Hence, the probability to observe barcode hopping in a dual-index RNA-Seq run is bounded by

$$p((b_{i7,d}, b_{i5,d}) \neq (b_{i7}, b_{i5}), (b_{i7,d}, b_{i5,d}) \in C) \leq \frac{1}{4} p((b_{i7,d}, b_{i5,d}) \not\in C, (b_{i7,d}, b_{i5,d}) \in B_{i7} \times B_{i5})^2 \quad (15)$$

If demultiplexing (i.e. assignment of sequencing reads with samples or index sequences) is followed by error correction, the probability of simultaneous barcode hopping of $b_{i7}$ and $b_{i5}$ increases. Equation (12), where $b_{i7/5,d}$ signifies sequences obtained by error correction, will, therefore, not be exact. However, since the upper bound for single-index barcode hopping is rather conservative we use (14), as an upper bound also in this case. In summary, an RNA-Seq run with UCDI can be used to

1. Detect but not correct barcode hopping of a single barcode after sequencing or error correction.
2. Derive an upper bound for the probability of single-index (14) and dual-index (15) barcode hopping after sequencing or error correction. The right-hand side of (14) is derived by demultiplexing with respect to all barcode tuples in $B_{i7} \times B_{i5}$ and calculating the ratio of $(b_{i7,d}, b_{i5,d}) \notin C$ in the set of all $(b_{i7,d}, b_{i5,d}) \in B_{i7} \times B_{i5}$ where $b_{i7/5,d}$ are the i7/5 indices after sequencing or error correction.

The present invention is further defined by the following examples, without being limited to these embodiments of the invention.

## Examples:

### Example 1: Generating nucleotide balanced ff-Levenshtein-7(8,10,12) barcode sets

To derive the barcode sets in this section we used Search Algorithm 4 described above in Section 4.1 with the fixed-frame Levenshtein distance as sequence distance. In step 1 of the algorithm we set $S = 5$, $E = 3$ and $n_1 = 8$, $n_2 = 10$ and $n_3 = 12$, resulting in an ff-Levenshtein-5(8,10,12) barcode set. We, further, chose for $i = 1, \ldots, 5$ and $j = 1,2,3$ inter-barcode distances with $d_{1,1} = 5$, $d_{i+1,1} = d_{i,1} - 1$ and $d_{i,j+1} = d_{i,j} + 1$ and error-correction distances $f_{i,j} = floor((d_{i,j} - 1)/2)$, where $floor(x)$ returns the highest integer smaller than *x*. These inter-barcode and error-correction distances can be found in Table 1. The set of potential barcodes *P* in Search Algorithm 4 was required to fulfill $P \neq \varnothing$. Hence, the first sequence satisfying the distance requirements in step 4 was added to the barcode set in step 6. Since the fixed-frame Levenshtein distance and the positional nucleotide distribution is independent of the sequence alphabet, we initially generated barcode sets from the alphabet 0, 1, 2, 3. To avoid barcode sequences consisting entirely of a single nucleotide as well as similar sequences we initialized our barcode set with $B_1 = \{(0, \ldots, 0), (1, \ldots, 1), (2, \ldots, 2), (3, \ldots, 3)\}$. After search completion these sequences were

removed from the barcode set. This resulted in nested barcode sets $EXT^j(B'_k)$ from which we selected nested barcode sets $EXT^j(B_i)$ with balanced nucleotide distribution and subsets $B_i$ with sizes 4, 8, 16, 24, 96, 768, 9216, hence producing an ff-Levenshtein-7(8,10,12) barcode set. Selection was performed using the algorithm in Section 4.3. The order in which elements of the complement $C^c$ were processed in step 4 of Search Algorithm 4 was chosen randomly. Thus, repetitions of the algorithm produced different results. In this manner we generated 245 barcode sets from which the final set was selected. Among the $EXT^j(B_i)$ with $|B_1| \geq 4$ we chose the nested barcode set whose nucleotide distributions $\delta(C_i,p,n)$ had minimal distance $D(\delta(C_i))$ from the uniform positional nucleotide distribution. As before, we set $C_i = EXT^E(B_i)$. In a final step we considered pairs of distinct mappings from alphabet 0,1,2,3 to *A, C, G, T,* producing pairs of barcode sets, and calculated the melting-temperature of all homo- and di-mers. In the end we selected the pair of barcode sets with the smallest melting temperatures and chose one of the barcode sets as "i7 indices" and the other as "i5 indices". The i5 and i7 notation refers to dual indexes added to separated locations of an oligonucleotide that is to be labelled (see Fig. 7 and ref. [5]). The number of elements and the inter-barcode distance of the nested subsets in the final ff-Levenshtein-7(8,10,12) set are given in Table 1 for nested sequences with 8, 10 and 12 nucleotides in length. Lengths 8 and 10 are nested in the larger sequence(s).

Table 1: Size and inter-barcode distance of subsets in ff-Leveshtein-7(8,10,12).

| | | | | 8nt | | 10nt | | 12nt | |
|---|---|---|---|---|---|---|---|---|---|
| $i$ | $\|B_i\|$ | $j$ | $\|B'_j\|$ | $d_{i,3}$ | $f_{i,3}$ | $d_{i,2}$ | $f_{i,2}$ | $d_{i,1}$ | $f_{i,1}$ |
| 1 | 4 | 1 | 6 | 5 | 2 | 6 | 2 | 7 | 3 |
| 2 | 8 | 2 | 25 | 4 | 1 | 5 | 2 | 6 | 2 |
| 3 | 16 | 2 | 25 | 4 | 1 | 5 | 2 | 6 | 2 |
| 4 | 24 | 2 | 25 | 4 | 1 | 5 | 2 | 6 | 2 |
| 5 | 96 | 3 | 104 | 3 | 1 | 4 | 1 | 5 | 2 |
| 6 | 768 | 4 | 835 | 2 | 0 | 3 | 1 | 4 | 1 |
| 7 | 9216 | 5 | 9545 | 1 | 0 | 2 | 0 | 3 | 1 |

The 104 sequences of the i7 and i5 indices in $B'_3$ of length 12 are contained in the sequence listing. SEQ ID NO: 1 to 104 are the i7 index sequences and SEQ ID NO: 105 to 208 are the i5 index sequences. The association between sequence numbers and barcode subsets $B_i$, $B'_j$ is given in Table 2. This clearly shows the nested structure of the subsets. Subsequences in these indices are the first 8 and 10 nucleotides, which exhibits the nested structure of subsequences in ff-Levenshtein-7(8,10,12).

For set i7:

| $i$ | $B_i$ | $j$ | $B'_j$ |
|---|---|---|---|
| 1 | SEQ ID NO: 1-4 | 1 | SEQ ID NO: 1-4,9,10 |
| 2 | SEQ ID NO: 1-8 | 2 | SEQ ID NO: 1-25 |
| 3 | SEQ ID NO: 1-16 | 2 | SEQ ID NO: 1-25 |
| 4 | SEQ ID NO: 1-24 | 2 | SEQ ID NO: 1-25 |
| 5 | SEQ ID NO: 1-96 | 3 | SEQ ID NO: 1-104 |

For set i5:

Table 2: Sequence numbers of barcodes in subsets.

| $i$ | $B_i$ | $j$ | $B'_j$ |
|---|---|---|---|
| 1 | SEQ ID NO: 105-108 | 1 | SEQ ID NO: 105-108, 113, 114 |
| 2 | SEQ ID NO: 105-112 | 2 | SEQ ID NO: 105-129 |
| 3 | SEQ ID NO: 105-120 | 2 | SEQ ID NO: 105-129 |
| 4 | SEQ ID NO: 105-128 | 2 | SEQ ID NO: 105-129 |

(continued)

| $i$ | $B_i$ | $j$ | $B'_j$ |
|---|---|---|---|
| 5 | SEQ ID NO: 105-200 | 3 | SEQ ID NO: 105-208 |

Figures 7, 8, 9 and 10 show the positional nucleotide distribution for $B_1$, $B_2$, $B_3$ and $B_4$. Here, the x-axis represents the barcode position p and the y-axis represents $\delta(C_i,p,n)$, the proportion of nucleotide $n$ at position p. Each of the 4 lines in these figures represents the proportion $\delta(C_i,p,n)$ for one of the 4 nucleotides $n$. For $B_1$, which contains 4 barcodes, $\delta(C_i,p,n)$ is uniform if at each position each nucleotide occurs in exactly one barcode. If $\delta(C_1,p,n)$ is not uniform in position p then at least one of the nucleotides $n$ is not contained in any of the 4 barcodes at position p and, therefore, $\delta(C_1,p,n) = 0$. Hence, Figure 8 shows that $\delta(C_1,p,n)$ is not uniform at 8 positions. In these positions $\delta(C_1,p,n) = 0$ only for a single nucleotide $n$ and the other nucleotides are, therefore, present. This is sufficient to obtain good quality scores on two-color Illumina sequencers which require the presence of A or C and, additionally, G or T [5] at each position. Figures 8, 9 and 10 show that for $B_2$, $B_3$ and $B_4$ all nucleotides are present at all positions, since lines $\delta(C_1,p,n)$ in these graphics are never zero. For $B_2$, which contains 8 barcodes, Figure 9 shows that at 8 positions one nucleotide occurs only once. For $B_3$, with $|B_3| = 16$, Figure 10 shows a single position where one nucleotide occurs only twice. Figure 11 shows for $B_4$, with $|B_4| = 24$, two positions with one nucleotide occurring four times and another position with two nucleotides occurring four times. These are the positions $p$ where $\delta(C_1,p,n)$ deviates most strongly from the uniform distribution. Overall, this shows that $\delta(C_i,p,n)$ approaches the uniform distribution with increasing $i$.

**Example 2: Cross-contamination in RNA-Seq runs with Hamming(6) and ff-Levenshtein-7(8,10,12) barcode sets**

For the experiments in this section we synthesized the 96 barcodes of length 12 in $B_5$ of the nucleotide balanced ff-Levenshtein-7(8,10.12) barcode set in Example 1. We further synthesized 96 barcodes of length 6 which had a minimal Hamming distance of 3. Hence, this set can correct one substitution. We synthesized both barcode sets as i5 and i7 indices and used them as unique dual indices (UDIs) to label 96 samples of commercially available universal human reference RNA (UHRR) in two dual-index RNA-Seq runs. UDIs are UCDIs with the additional requirement that $b_{i7} \neq b_{i5}$. Subsequently, we demultiplexed each run with respect to the entire 96 i5/i7 barcode tuples of the respective UDIs and estimated barcode-hopping rates as well as rates of cross-contamination after error correction. We further calculated the purity, i.e. the fraction of $(b_{i7,d}, b_{i5,d}) \in B_{i7} \times B_{i5}$. In the case of the ff-Levenshtein-7(8,10,12) set we performed this analysis for all barcode lengths 8, 10 and 12. The results can be found in Table 3. This shows that without error correction the error-rate for $B_5$ of ff-Levenshtein-7(8,10,12) is almost identical for length 8, 10 and 12 at 0.01%, while purity is highest for length 8, at 93.028%, and lowest for length 12, at 90.913%. The decrease in purity with increasing barcode length is due to the fact that longer sequences have a higher chance to contain an error. In comparison to ff-Levenshtein-7(8,10,12), for Hamming(6) the error-rate is considerably higher at 0.244%. This increase over ff-Levenshtein-7(8,10,12) by a factor of 24 is not just the result of a shorter barcode length but also due to the different distance used for the barcode design. In comparison to the ff-Levenshtein distance, barcode sets designed with the Hamming distance do not guarantee a sensible inter-barcode distance after insertions and deletions. This can also be seen when correcting a single error for Hamming(6), which increases the error rate by a factor of around 7 to 1.5%. In comparison, correcting a single error for $B_5$ of ff-Levenshtein-7(8,10,12) the error rate at length 12 remains unchanged at 0.01%, while purity increases to 97.013%. For ff-Levenshtein-7(8,10,12) and length 10 the error rate increases slightly, while for ff-Levenshtein-7(8,10,12) and length 8 the error rate increases by a factor of 10 to 0.1%. This shows that if one error is to be corrected using a barcode length of at least 10 is advisable for ff-Levenshtein-7 (8, 10, 12). On the other hand, if a barcode length of 12 has to be chosen it is advisable to perform error correction since this will increase purity to the same level as for barcode lengths 8 and 10. Correcting 2 errors, which is only possible for ff-Levenshtein(12), leads only to a small increase in purity at the expense of a more then two-fold increase in error rate to 0.024%. Hence, correcting two errors with ff-Levenshtein-7(8,10,12) and length 12 is not advisable. Overall, the results in the experiments in Table 3 show that cross-contamination for $B_5$ of ff-Levenshtein-7(8,10,12) is considerably lower than for Hamming(6) while purity increases. The results, further, show that all barcode lengths of ff-Levenshtein-7(8,10,12) can be used for multiplexing samples in an RNA-seq run.

Table 3: Error and purity (%) for Hamming(6) (H(6)) and ff-Levenshtein-7(8,10,12) with length 8 (ff-L(8)), 10 (ff-L(10)) and 12 (ff-L(12)) without error correction and with correction of 1 error (1c) and 2 errors (2c).

| | H(6) | H(6) 1c | ff-L(8) | ff-L(10) | ff-L(12) | ff-L(8) 1c | ff-L(10) 1c | ff-L(12) 1c | ff-L(12) 2c |
|---|---|---|---|---|---|---|---|---|---|
| Error | 0.244 | 1.564 | 0.010 | 0.009 | 0.009 | 0.110 | 0.015 | 0.010 | 0.024 |
| Purity | 90.025 | 95.711 | 93.028 | 91.953 | 90.913 | 97.590 | 97.251 | 97.013 | 97.768 |

**Example 3: Quantifying various types of low-level cross-contamination with ff-Levenshtein-5(8,10,12)**

The experiments in this section evaluated cross-contamination at different stages of index synthesis, library preparation and sequencing. To measure the low levels of cross-contamination expected, we chose 12 barcodes from the 25 barcodes in $B_2$, of ff-Levenshtein-5(8,10,12) in Example 1. The selected barcode set contained all barcodes in $B_1$, and, as a result, 6 barcodes had an ff-Levenshtein distance of 7 for barcode length 12, while all 12 barcodes had an ff-Levenshtein distance of 6. We divided this barcode set into 3 sets of 4 barcodes which were synthesized by 3 oligonucleotide synthesis providers as i5 and i7 barcodes. We used the barcodes as unique dual indices with $b_{i7} = b_{i5}$. Such sets of unique dual-indices are also called unique dual-matched indices (UDMIs) [5]. In our experiment we labeled 9 samples of the UHRR in Section 3.2 with 3 UDMIs of each synthesis provider. The remaining 3 UDMIs, one for each provider, were never touched. This experimental design allows to estimate cross-contamination at the synthesis provider site, since detection of a left-out barcode after demultiplexing shows that this barcode ended up in the wrong tube before delivery. As in Example 2, we demultiplexed with respect to all tuples of the 96 barcodes in $B_5$ of ff-Levenshtein-7(8, 10, 12) . This gave us a count matrix with rows and columns labeled by $b_{i7}$ and $b_{i5}$. Different types of cross-contamination correspond to different regions in this matrix, which are visualized in Figure 12. Elements in region C correspond to barcode tuples ($b_{i7}$, $b_{i5}$), where at least one of $b_{i7}$, $b_{i5}$ has not appeared at any stage of the experiment. Counts in region C, therefore, quantify the frequency with which the RNA-Seq run randomly generates an i5 or i7 index in barcode set $B_5$. Region B contains barcode tuples ($b_{i7}$, $b_{i5}$) where both $b_{i7}$ and $b_{i5}$ were synthesized but where at least one of $b_{i7}$, $b_{i5}$ was never used in the experiment. Counts in region B, therefore, quantify the cumulative random cross-contamination and the cross-contamination at the synthesis provider site. Region A contains tuples ($b_{i7}$, $b_{i5}$) where both $b_{i7}$ and $b_{i5}$ were synthesized and used in the experiment. Off-diagonal elements in region A, therefore, quantify the cumulative random, site dependent and experimental cross-contamination. Experimental cross-contamination contains, amongst others, cross-contamination due to handling errors, laboratory conditions and provider dependent experimental errors. The latter can, for instance, be the result of the instability of synthesized sequences during sequencing. Differences in synthesis provider dependent experimental error are reflected by differences in the off-diagonal counts in regions P1, P2 and P3, which contain barcode tuples ($b_{i7}$, $b_{i5}$) generated by provider 1, 2 and 3. From the regions in the count matrix in Figure 12 we derived the amounts of cross-contamination in Table 4. Due to small levels of cross-contamination, values in Table 4 are given in parts per million. The rows in Table 4 are labeled with the region in which the cross-contamination was measured. Row label "C diag" stands for the amount of cross-contamination in the diagonal of region "C". Label "A-nonP" stands for the provider independent experimental cross-contamination. We estimate the latter by subtracting the provider dependent from the overall experimental cross-contamination. To avoid underestimation of the provider independent experimental cross-contamination we assume that the smallest cross-contamination measured for any provider is entirely the result of provider independent factors. On the other hand, we assume that differences between the provider dependent experimental cross-contamination are entirely the result of provider dependent factors. In Table 4, therefore, we calculate the provider independent experimental cross-contamination as $A - nonP = A - (P1 + P3 - 2P2)$.

Table 4: Amount (parts per million) of different types of synthesis provider dependent cross-contamination.

| | ff-L(8) | ff-L (10) | ff-L(12) |
|---|---|---|---|
| C | 52.565 | 0.072 | 0.000 |
| C diag | 16.741 | 0.000 | 0.000 |
| B | 0.019 | 0.003 | 0.003 |
| A | 18.840 | 14.599 | 11.904 |
| P1 | 1.367 | 1.197 | 1.095 |
| P2 | 0.382 | 0.339 | 0.283 |
| P3 | 5.256 | 3.857 | 3.122 |
| A-nonP | 12.981 | 10.223 | 8.253 |
| Total | 71.424 | 14.674 | 11.907 |

Table 4 shows that the total cross-contamination increases significantly when reducing the barcode length to 8. For barcode length 10 and 12 overall cross-contamination is smaller by a factor of around 5 and 6, respectively. The main contribution to this increase comes from the large random error (C) for barcode length 8. Table 4 shows, further, that the overall cross-contamination at the provider site (B) is negligible. However, experimental cross-contamination differs noticeably between providers. In comparison to provider 2 (P2), experimental cross-contamination for provider 1 (P1) and

3 (P3) is higher by a factor of around 4 and 11, respectively. The provider independent experimental cross-contamination (A-nonP) is, for all barcode lengths, close to 69% of the total minus the random cross-contamination indicating that 69% of non-random cross-contamination in this experiment is contributed by provider independent sources.

Overall, the results in this example show that the ff-Levenshtein-5(8,10,12) barcode set can be used with barcode lengths 8, 10 and 12 to quantify low levels of cross-contamination. This is a necessary prerequisite for identifying and reducing different sources of cross-contamination.

### References

[1] Buschmann and Bystrykh. BMC Bioinformatics, 14:272, 2013
[2] Conway and Sloane. IEEE Trans. Inf. Theor., 32(3):337-348, 1986
[3] Hawkins et al. PNAS, 115(27): E6217-E6226, 2018
[4] WO 2018/204423 A1
[5] MacConaill et al. BMC Genomics, 19(1):30-30, 2018
[6] WO 2018/136248 A1
[7] WO 2018/204423 A1
[8] WO 2011/100617 A1


### Claims

**1.** A computer-implemented method of generating a set of oligonucleotides comprising a plurality of subsets of oligonucleotides with a subset of index sequences comprising the steps of

generating a first subset of oligonucleotides with index sequences with a first sequence distance to each other within the first subset, wherein a sequence distance is a quantified amount of sequence changes that transforms one sequence into another or a monotonically decreasing function of a probability of sequence changes that transforms one sequence into another,
generating a second subset by including the first subset and adding further oligonucleotides with index sequences with a second sequence distance to each other within the second subset, which second sequence distance is a lower sequence distance than the first sequence distance, whereby the second subset comprises oligonucleotides with index sequences that are not part of the first subset.

**2.** The method of claim 1, wherein generating a first and/or second subset comprises selecting index sequences that comprise truncated index sequences and the truncated index sequences of at least one subset differ at least by a non-zero number of sequence changes from each other truncated index sequence within said subset.

**3.** The method of claim 1 or 2, wherein generating a subset comprises selecting index sequences by adding an index sequence candidate and evaluating the sequence distance of the index distance candidate to all other pre-existing index sequences in the subset; and adding the index sequence candidate to the index sequences of the subset if it fulfils a pre-set sequence distance requirement.

**4.** The method of claim 1, 2 or 3, wherein an index sequence candidate is selected from a pool of index sequence candidates, wherein members of the pool of index sequence candidates fulfil a pre-set sequence distance requirement to each other member of the pool, and wherein an index sequence candidate of the pool is added to the index sequences of the subset, when the sum of the absolute values of the differences of the frequency of each nucleotide type of the genetic code at each position to 0.25 is the lowest for the index sequence candidate as compared to the other index sequence candidates of the pool.

**5.** The method of any one of claims 1 to 4, comprising generating a plurality of subset candidates each with a given amount of members as in the methods of claims 1 to 4 and selecting a subset candidate as subset, when said subset candidate has the lowest average over all index sequences per respective subset candidate of the sum of the absolute values of differences of the frequency of each nucleotide type of the genetic code at each position to 0.25; or comprising generating a plurality of subset candidates each with a given amount of members as in the methods of claims 1 to 4 and selecting a subset candidate as subset, wherein said subset candidate is selected by exclusion of other subset candidates,

wherein a subset candidate is excluded when in a method that comprises adding index sequence candidates from a pool of index sequence candidates to the subset candidate, and optionally further adding comparative index sequences, the subset candidate has a higher average over all its index sequences sum of absolute values of differences of the frequency of each nucleotide type of the genetic code at each position to 0.25 as compared to another subset or subset candidate.

**6.** A computer-implemented method of assigning sequencing reads to a sample of oligonucleotides comprising the steps of

a) obtaining sample oligonucleotides from a plurality of samples,
b) generating a set of oligonucleotides according to any one of claims 1 to 5 and selecting a subset of oligonucleotide index sequences from the set of oligonucleotides;
wherein a subset is selected over another subset based on a higher sequence distance of the index sequences to each other within the selected subset; wherein a sequence distance is a quantified amount of sequence changes that transforms one sequence into another or a monotonically decreasing function of a probability of sequence changes that transforms one sequence into another, and wherein the selected subset has at least as many different index sequences as the number of samples of step a),
c) adding index sequences from said subset to each sample oligonucleotide wherein the index sequences are indicative of the sample,
d) determining the sequence of the sample oligonucleotides or fragments of sample oligonucleotides and determining the index sequence,
e) assigning an obtained read sequence to a sample based on the determined index sequence or based on the index sequence which has the lowest sequence distance to the determined index sequence, wherein if two or more index sequence have the same lowest distance then said obtained read is discarded; wherein optionally the sequence distance does not exceed a pre-set criterion value.

**Patentansprüche**

**1.** Ein computerimplementiertes Verfahren zur Erzeugung eines Satzes von Oligonukleotiden, umfassend eine Vielzahl von Untermengen von Oligonukleotiden mit einer Untermenge von Indexsequenzen, umfassend die Schritte

Erzeugen einer ersten Untermenge von Oligonukleotiden mit Indexsequenzen mit einem ersten Sequenzabstand zueinander innerhalb der ersten Untermenge, wobei ein Sequenzabstand eine quantifizierte Menge von Sequenzänderungen ist, die eine Sequenz in eine andere umwandelt, oder eine monoton abnehmende Funktion einer Wahrscheinlichkeit von Sequenzänderungen ist, die eine Sequenz in eine andere umwandelt,
Erzeugen einer zweiten Untermenge durch Einschluss der ersten Untermenge und Hinzufügen weiterer Oligonukleotide mit Indexsequenzen mit einem zweiten Sequenzabstand zueinander innerhalb der zweiten Untermenge, wobei der zweite Sequenzabstand ein geringerer Sequenzabstand als der erste Sequenzabstand ist, wodurch die zweite Untermenge Oligonukleotide mit Indexsequenzen umfasst, die nicht Teil der ersten Untermenge sind.

**2.** Das Verfahren nach Anspruch 1, wobei das Erzeugen einer ersten und/oder zweiten Untermenge das Auswählen von Indexsequenzen umfasst, die verkürzte Indexsequenzen umfassen, und die verkürzten Indexsequenzen von mindestens einer Untermenge sich mindestens um eine von null verschiedene Anzahl von Sequenzänderungen von jeder anderen verkürzten Indexsequenz innerhalb dieser Untermenge unterscheiden.

**3.** Das Verfahren nach Anspruch 1 oder 2, wobei das Erzeugen einer Untermenge das Auswählen von Indexsequenzen durch Hinzufügen eines Indexsequenzkandidaten und Bewerten des Sequenzabstands des Indexabstandskandidaten zu allen anderen bereits vorhandenen Indexsequenzen in der Untermenge umfasst; und Hinzufügen des Indexsequenzkandidaten zu den Indexsequenzen der Untermenge, wenn er eine vorgegebene Sequenzabstandsanforderung erfüllt.

**4.** Das Verfahren nach Anspruch 1, 2 oder 3, wobei ein Indexsequenzkandidat aus einem Pool von Indexsequenzkandidaten ausgewählt wird, wobei Mitglieder des Pools von Indexsequenzkandidaten eine vorgegebene Sequenzabstandsanforderung zu jedem anderen Mitglied des Pools erfüllen, und wobei ein Indexsequenzkandidat des Pools zu den Indexsequenzen der Untermenge hinzugefügt wird, wenn die Summe der Absolutwerte der Differenzen der Häufigkeit jedes Nukleotidtyps des genetischen Codes an jeder Position zu 0,25 für den Indexsequenzkandidaten am

niedrigsten ist im Vergleich zu den anderen Indexsequenzkandidaten des Pools.

**5.** Das Verfahren nach einem der Ansprüche 1 bis 4, umfassend das Erzeugen einer Vielzahl von Untermengenkandidaten, jeder mit einer gegebenen Anzahl von Mitgliedern wie in den Verfahren der Ansprüche 1 bis 4, und Auswählen eines Untermengenkandidaten als Untermenge, wenn dieser Untermengenkandidat den niedrigsten Durchschnitt über alle Indexsequenzen pro jeweiligem Untermengenkandidaten der Summe der Absolutwerte der Differenzen der Häufigkeit jedes Nukleotidtyps des genetischen Codes an jeder Position zu 0,25 hat;
oder
umfassend das Erzeugen einer Vielzahl von Untermengenkandidaten, jeder mit einer gegebenen Anzahl von Mitgliedern wie in den Verfahren der Ansprüche 1 bis 4, und Auswählen eines Untermengenkandidaten als Untermenge, wobei dieser Untermengenkandidat durch Ausschluss anderer Untermengenkandidaten ausgewählt wird, wobei ein Untermengenkandidat ausgeschlossen wird, wenn in einem Verfahren, das das Hinzufügen von Indexsequenzkandidaten aus einem Pool von Indexsequenzkandidaten zu dem Untermengenkandidaten umfasst und optional das weitere Hinzufügen von Vergleichsindexsequenzen, der Untermengenkandidat einen höheren Durchschnitt über alle seine Indexsequenzen der Summe der Absolutwerte der Differenzen der Häufigkeit jedes Nukleotidtyps des genetischen Codes an jeder Position zu 0,25 hat im Vergleich zu einer anderen Untermenge oder einem anderen Untermengenkandidaten.

**6.** Ein computerimplementiertes Verfahren zur Zuordnung von Sequenzierungsreads zu einer Probe von Oligonukleotiden, umfassend die Schritte

a) Erhalten von Probenoligonukleotiden aus einer Vielzahl von Proben,
b) Erzeugen eines Satzes von Oligonukleotiden nach einem der Ansprüche 1 bis 5 und Auswählen einer Untermenge von Oligonukleotid-Indexsequenzen aus dem Satz von Oligonukleotiden;
wobei eine Untermenge gegenüber einer anderen Untermenge basierend auf einem höheren Sequenzabstand der Indexsequenzen zueinander innerhalb der ausgewählten Untermenge ausgewählt wird; wobei ein Sequenzabstand eine quantifizierte Menge von Sequenzänderungen ist, die eine Sequenz in eine andere umwandelt, oder eine monoton abnehmende Funktion einer Wahrscheinlichkeit von Sequenzänderungen ist, die eine Sequenz in eine andere umwandelt, und wobei die ausgewählte Untermenge mindestens so viele verschiedene Indexsequenzen hat wie die Anzahl der Proben von Schritt a),
c) Hinzufügen von Indexsequenzen aus dieser Untermenge zu jedem Probenoligonukleotid, wobei die Indexsequenzen für die Probe indikativ sind,
d) Bestimmen der Sequenz der Probenoligonukleotide oder Fragmente von Probenoligonukleotiden und Bestimmen der Indexsequenz,
e) Zuordnen einer erhaltenen Read-Sequenz zu einer Probe basierend auf der bestimmten Indexsequenz oder basierend auf der Indexsequenz, die den niedrigsten Sequenzabstand zu der bestimmten Indexsequenz aufweist, wobei, wenn zwei oder mehr Indexsequenzen denselben niedrigsten Abstand aufweisen, dieser erhaltene Read verworfen wird; wobei optional der Sequenzabstand einen vorgegebenen Kriteriumswert nicht überschreitet.

## Revendications

**1.** Méthode informatisée de génération d'un ensemble d'oligonucléotides comprenant une pluralité de sous-ensembles d'oligonucléotides avec un sous-ensemble de séquences d'index, comprenant les étapes de

générer un premier sous-ensemble d'oligonucléotides avec des séquences d'index ayant une première distance de séquence entre elles au sein du premier sous-ensemble, la distance de séquence étant une quantité mesurée de modifications de séquence transformant une séquence en une autre ou une fonction strictement décroissante de la probabilité de modifications de séquence transformant une séquence en une autre,
générer un deuxième sous-ensemble en incluant le premier sous-ensemble et en ajoutant des oligonucléotides supplémentaires avec des séquences d'index ayant une deuxième distance de séquence entre elles au sein du deuxième sous-ensemble, cette deuxième distance de séquence étant une distance inférieure à la première distance de séquence, de sorte que le deuxième sous-ensemble comprenne des oligonucléotides avec des séquences d'index qui ne font pas partie du premier sous-ensemble.

**2.** Méthode selon la revendication 1, dans laquelle la génération d'un premier et/ou deuxième sous-ensemble comprend la sélection de séquences d'index comprenant des séquences d'index tronquées, et les séquences d'index tronquées

d'au moins un sous-ensemble diffèrent entre elles par au moins un nombre non nul de modifications de séquence.

**3.** Méthode selon la revendication 1 ou 2, dans laquelle la génération d'un sous-ensemble comprend la sélection de séquences d'index en ajoutant un candidat de séquence d'index et en évaluant la distance de séquence du candidat de séquence d'index par rapport à toutes les autres séquences d'index préexistantes dans le sous-ensemble ; et en ajoutant le candidat de séquence d'index aux séquences d'index du sous-ensemble s'il satisfait à un critère de distance de séquence prédéfini.

**4.** Méthode selon l'une quelconque des revendications 1 à 3, dans laquelle un candidat de séquence d'index est sélectionné à partir d'un ensemble de séquences de candidats de séquences d'index, les membres de l'ensemble de candidats de séquences d'index satisfaisant à un critère de distance de séquence prédéfini les uns par rapport aux autres, et dans laquelle un candidat de séquence d'index de l'ensemble est ajouté aux séquences d'index du sous-ensemble lorsque la somme des valeurs absolues des différences de la fréquence de chaque type de nucléotide du code génétique à chaque position par rapport à 0,25 est la plus faible pour le candidat de séquence d'index par rapport aux autres candidats de séquences d'index de l'ensemble.

**5.** Méthode selon l'une quelconque des revendications 1 à 4, comprenant la génération d'une pluralité de candidats de sous-ensembles, chacun ayant un nombre donné de membres selon les méthodes des revendications 1 à 4, et la sélection d'un candidat de sous-ensemble comme sous-ensemble lorsque ledit candidat de sous-ensemble a la moyenne la plus faible sur toutes les séquences d'index par rapport à chaque candidat de sous-ensemble de la somme des valeurs absolues des différences de la fréquence de chaque type de nucléotide du code génétique à chaque position par rapport à 0,25 ;
ou
comprenant la génération d'une pluralité de candidats de sous-ensembles, chacun ayant un nombre donné de membres selon les méthodes des revendications 1 à 4, et la sélection d'un candidat de sous-ensemble comme sous-ensemble,
dans lequel ledit candidat de sous-ensemble est sélectionné par exclusion des autres candidats de sous-ensembles, un candidat de sous-ensemble étant exclu lorsqu'une méthode comprenant l'ajout de candidats de séquences d'index à partir d'un ensemble de séquences de candidats de séquences d'index au candidat de sous-ensemble, et éventuellement l'ajout de séquences d'index comparatives, le candidat de sous-ensemble a une moyenne plus élevée sur toutes ses séquences d'index de la somme des valeurs absolues des différences de la fréquence de chaque type de nucléotide du code génétique à chaque position par rapport à 0,25 par rapport à un autre sous-ensemble ou candidat de sous-ensemble.

**6.** Méthode informatisée d'affectation de lectures de séquençage à un échantillon d'oligonucléotides, comprenant les étapes de :

a) obtenir des oligonucléotides provenant d'une pluralité d'échantillon à partir d'une pluralité d'échantillons,
b) générer un ensemble d'oligonucléotides selon l'une quelconque des revendications 1 à 5 et sélectionner un sous-ensemble de séquences d'index d'oligonucléotides à partir de l'ensemble d'oligonucléotides ;
dans lequel un sous-ensemble est sélectionné par rapport à un autre sous-ensemble sur la base d'une distance de séquence plus élevée des séquences d'index entre elles au sein du sous-ensemble sélectionné ; la distance de séquence étant une quantité mesurée de modifications de séquence transformant une séquence en une autre ou une fonction monotone décroissante de la probabilité de modifications de séquence transformant une séquence en une autre, et dans lequel le sous-ensemble sélectionné comporte au moins autant de séquences d'index différentes que le nombre d'échantillons de l'étape a),
c) ajouter des séquences d'index issues du sous-ensemble à chaque oligonucléotide d'échantillon, les séquences d'index étant indicatives de l'échantillon,
d) déterminer la séquence des oligonucléotides d'échantillon ou des fragments d'oligonucléotides d'échantillon et déterminer la séquence d'index,
e) affecter une lecture à un échantillon sur la base de la séquence d'index déterminée ou sur la base de la séquence d'index qui a la distance de séquence la plus faible par rapport à la séquence d'index déterminée, dans lequel si deux ou plusieurs séquences d'index ont la même distance la plus faible, ladite lecture obtenue est rejetée ; éventuellement, la distance de séquence ne dépasse pas une valeur seuil prédéfinie.

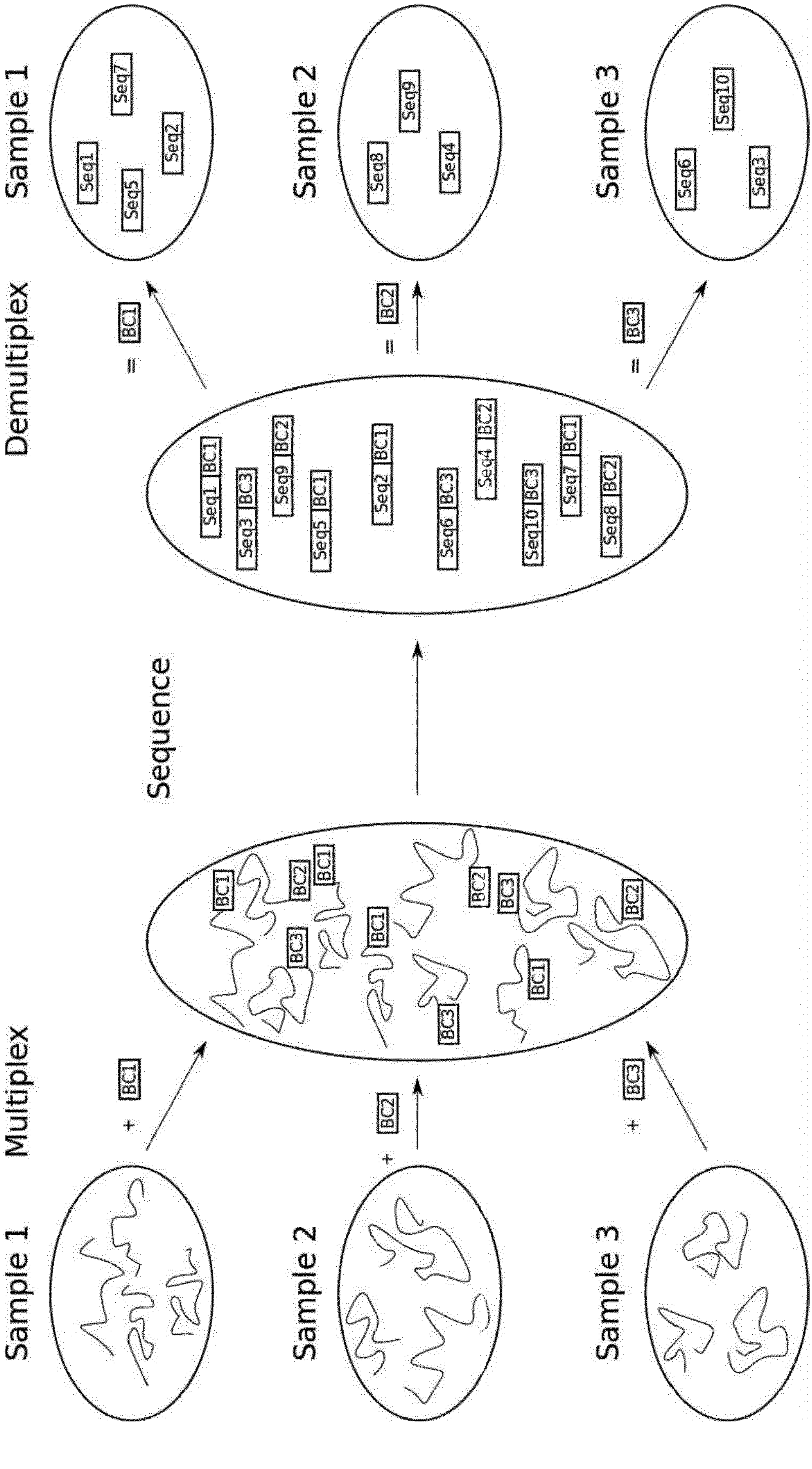
Sample 1
Sample 2
Sample 3
Multiplex
+ BC1
+ BC2
+ BC3
Sequence
Demultiplex
= BC1
= BC2
= BC3
Seq1 BC1
Seq3 BC3
Seq9 BC2
Seq5 BC1
Seq2 BC1
Seq6 BC3
Seq4 BC2
Seq10 BC3
Seq7 BC1
Seq8 BC2
Seq1
Seq5
Seq7
Seq2
Seq8
Seq9
Seq4
Seq6
Seq10
Seq3

Figure 1

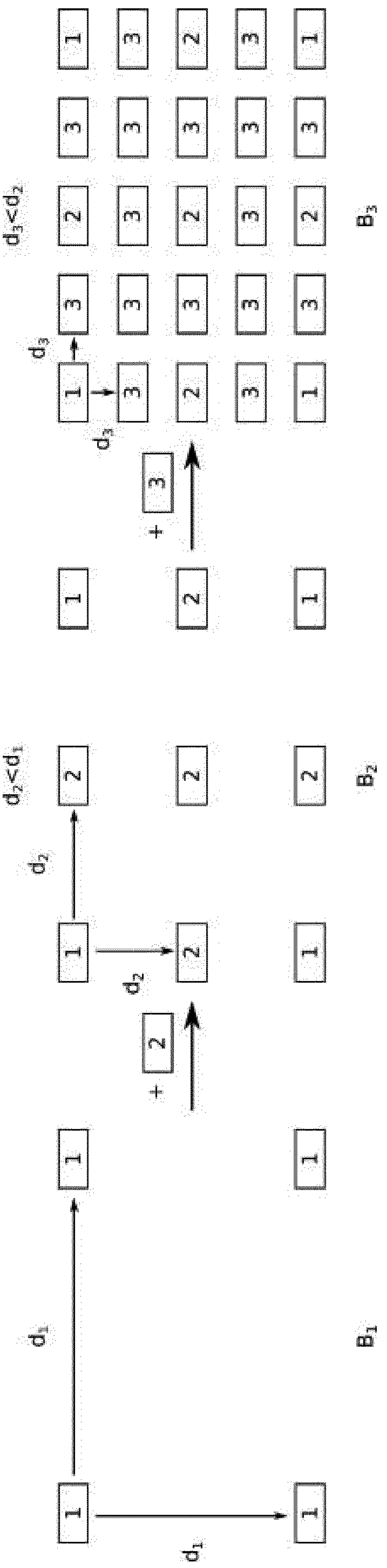


Figure 2

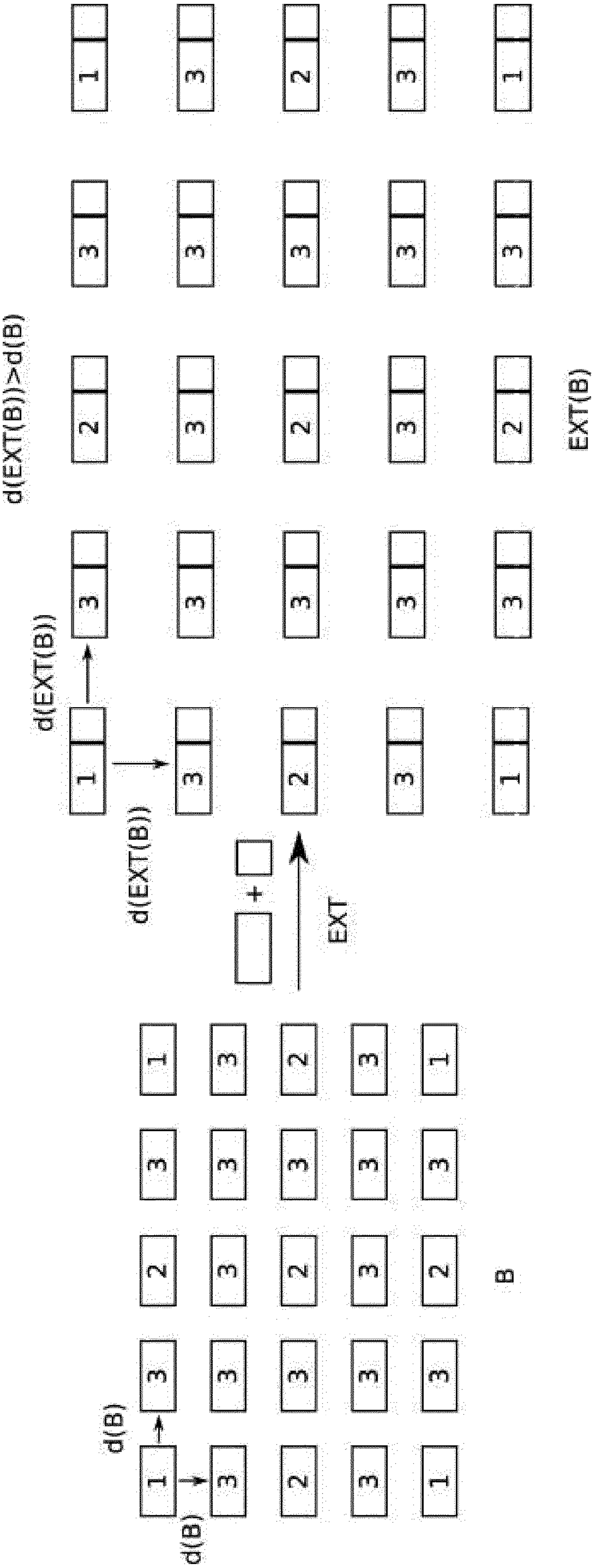


Figure 3

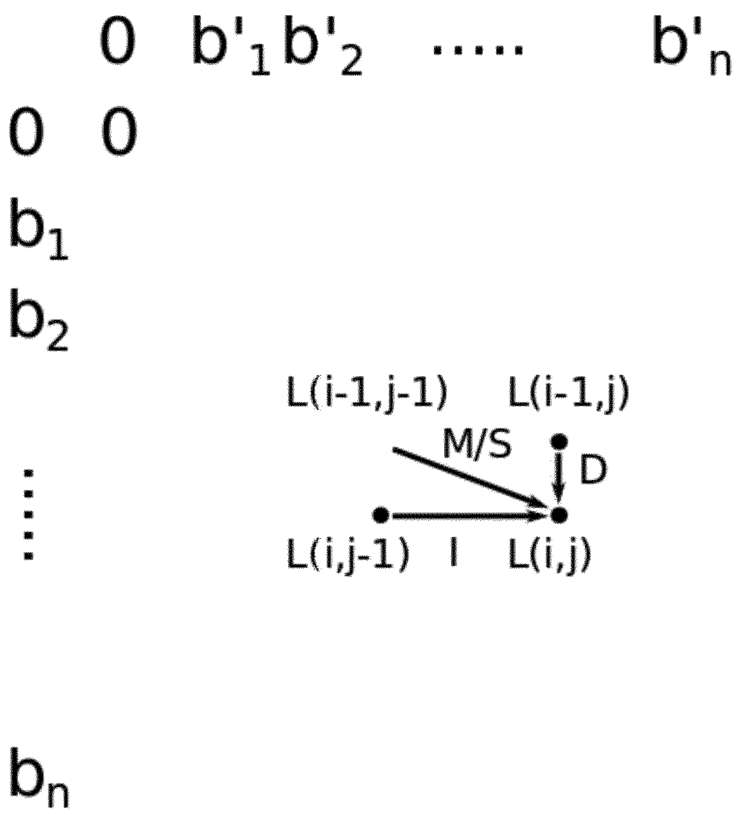


Figure 4

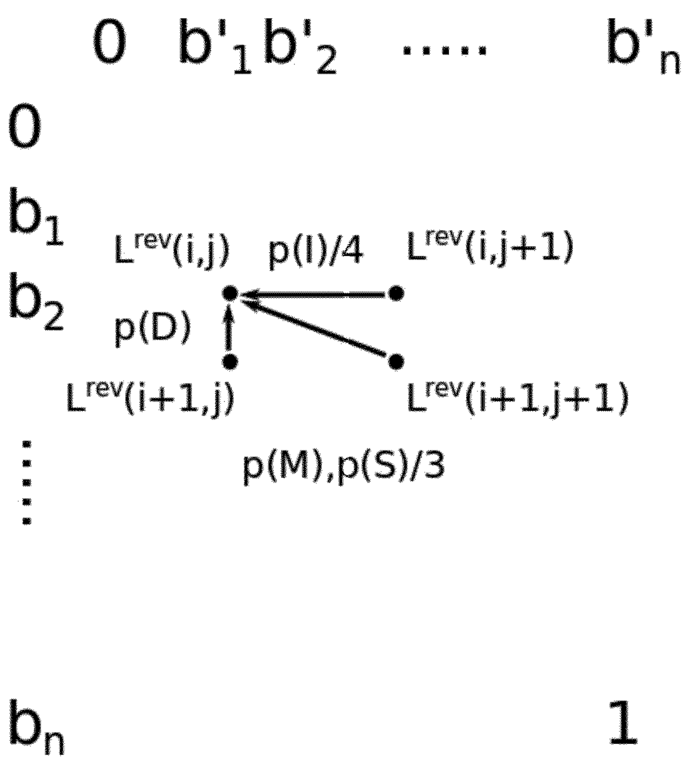


Figure 5

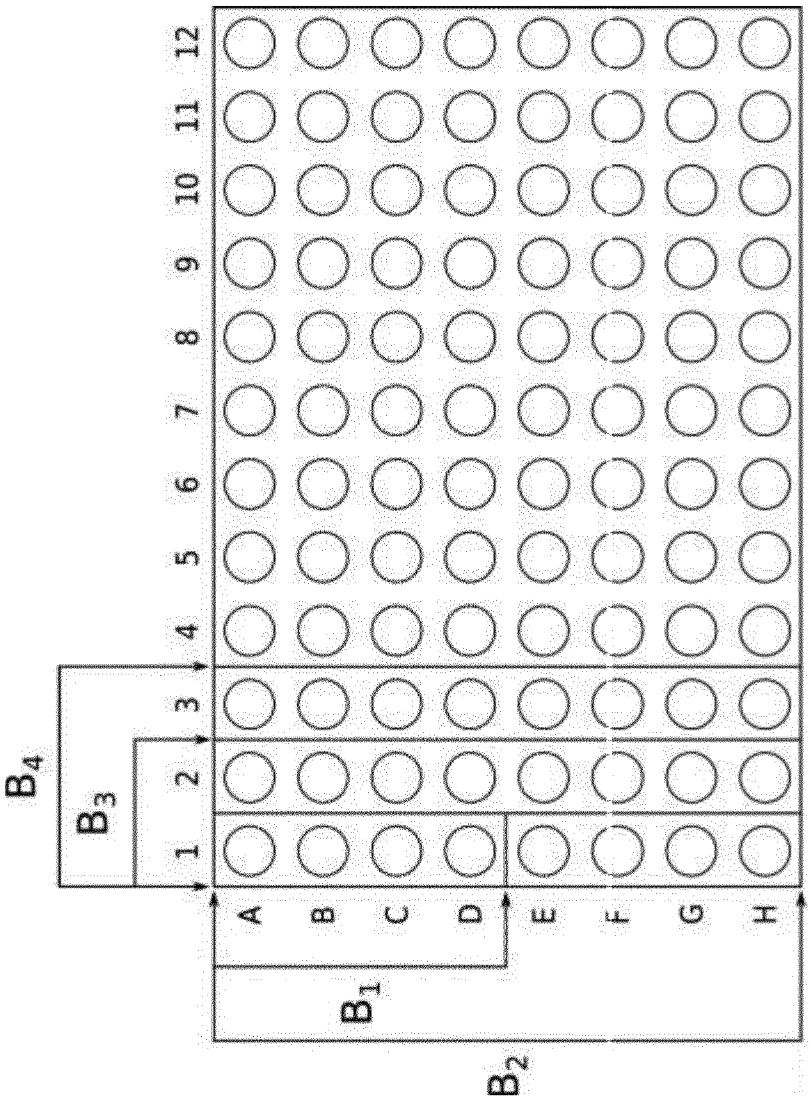


Figure 6

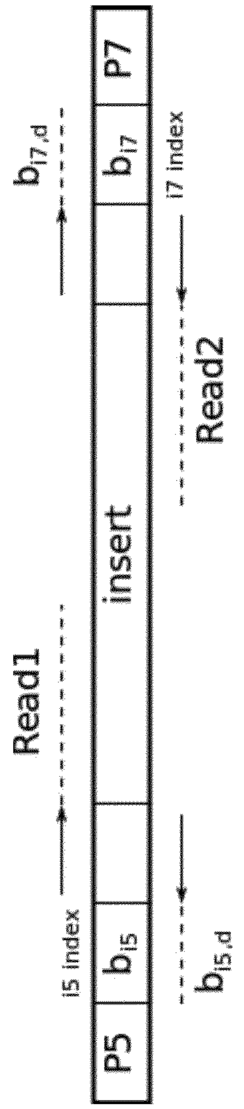


Figure 7

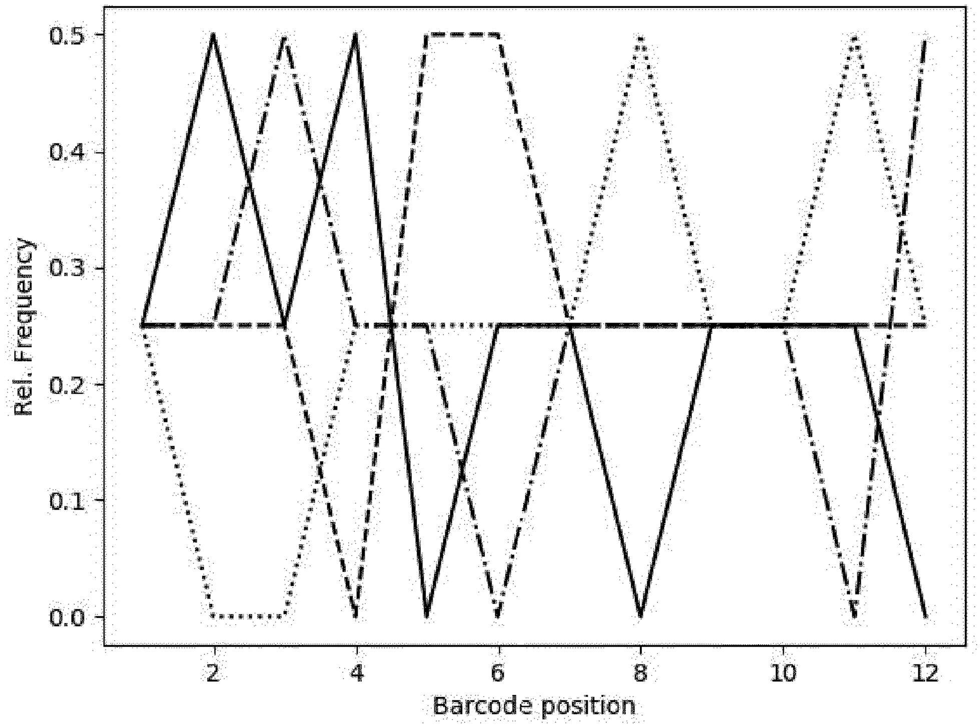


Figure 8

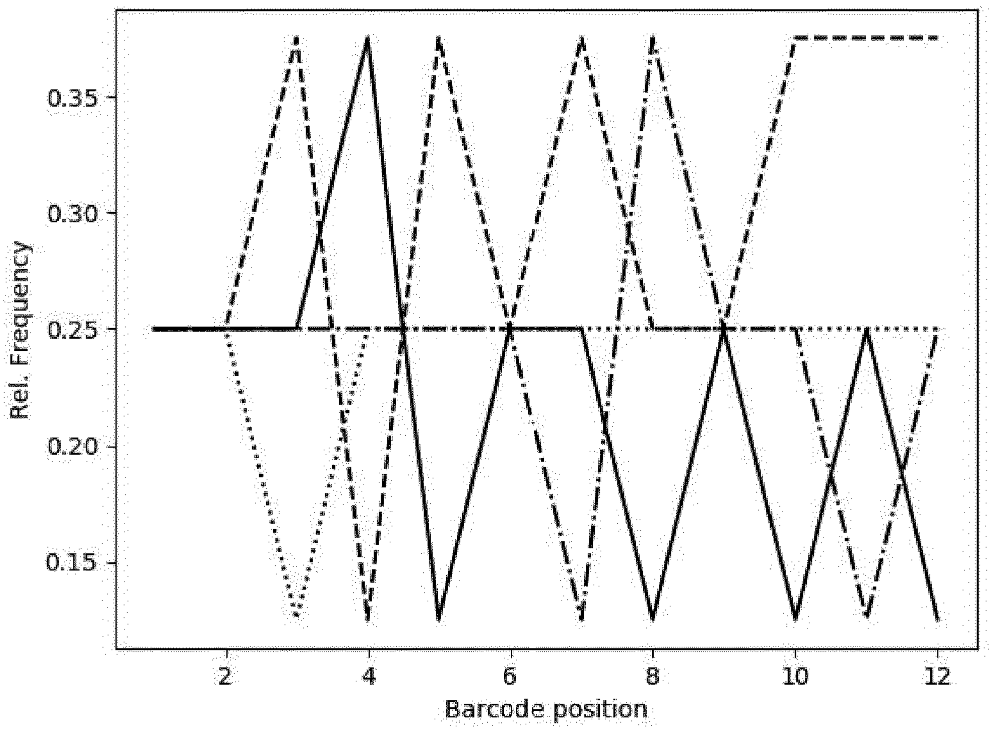


Figure 9

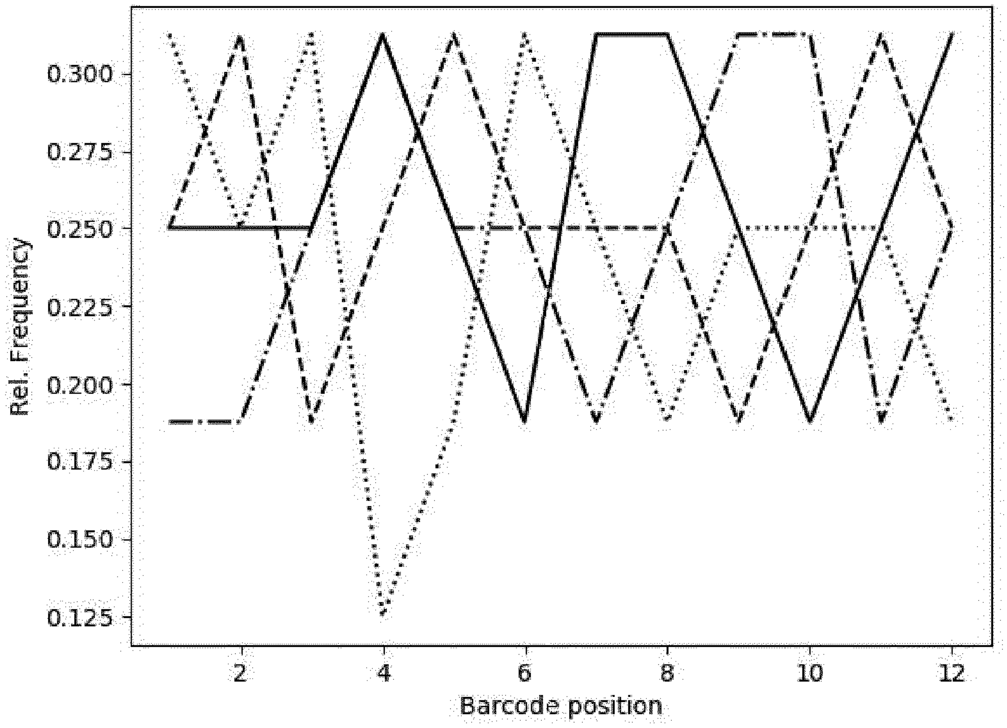


Figure 10

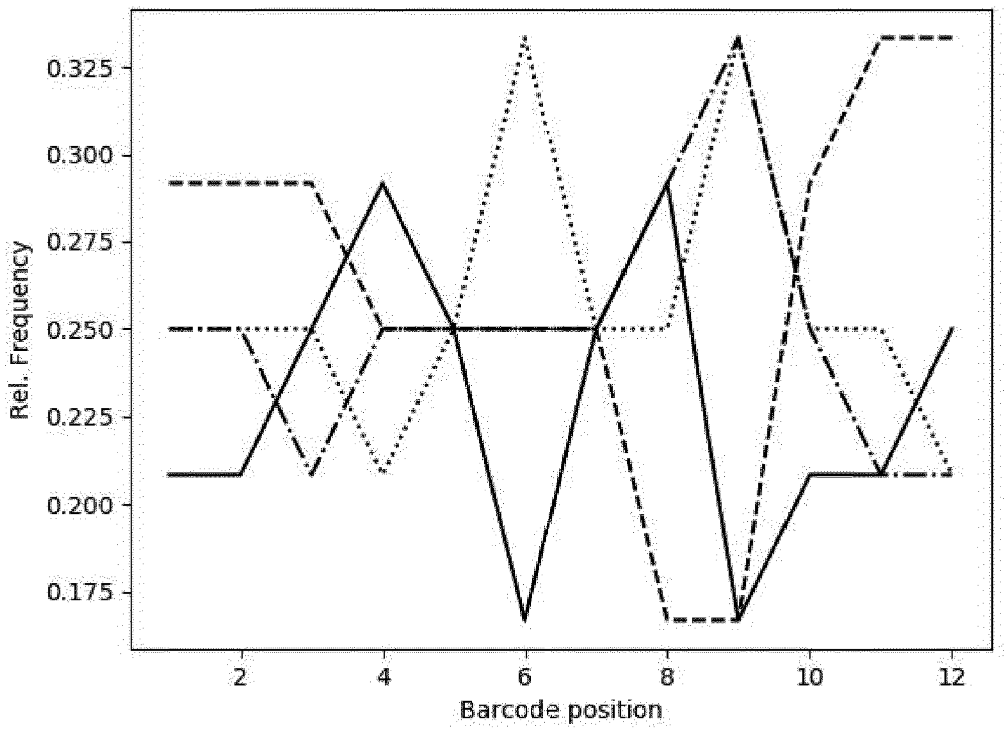


Figure 11

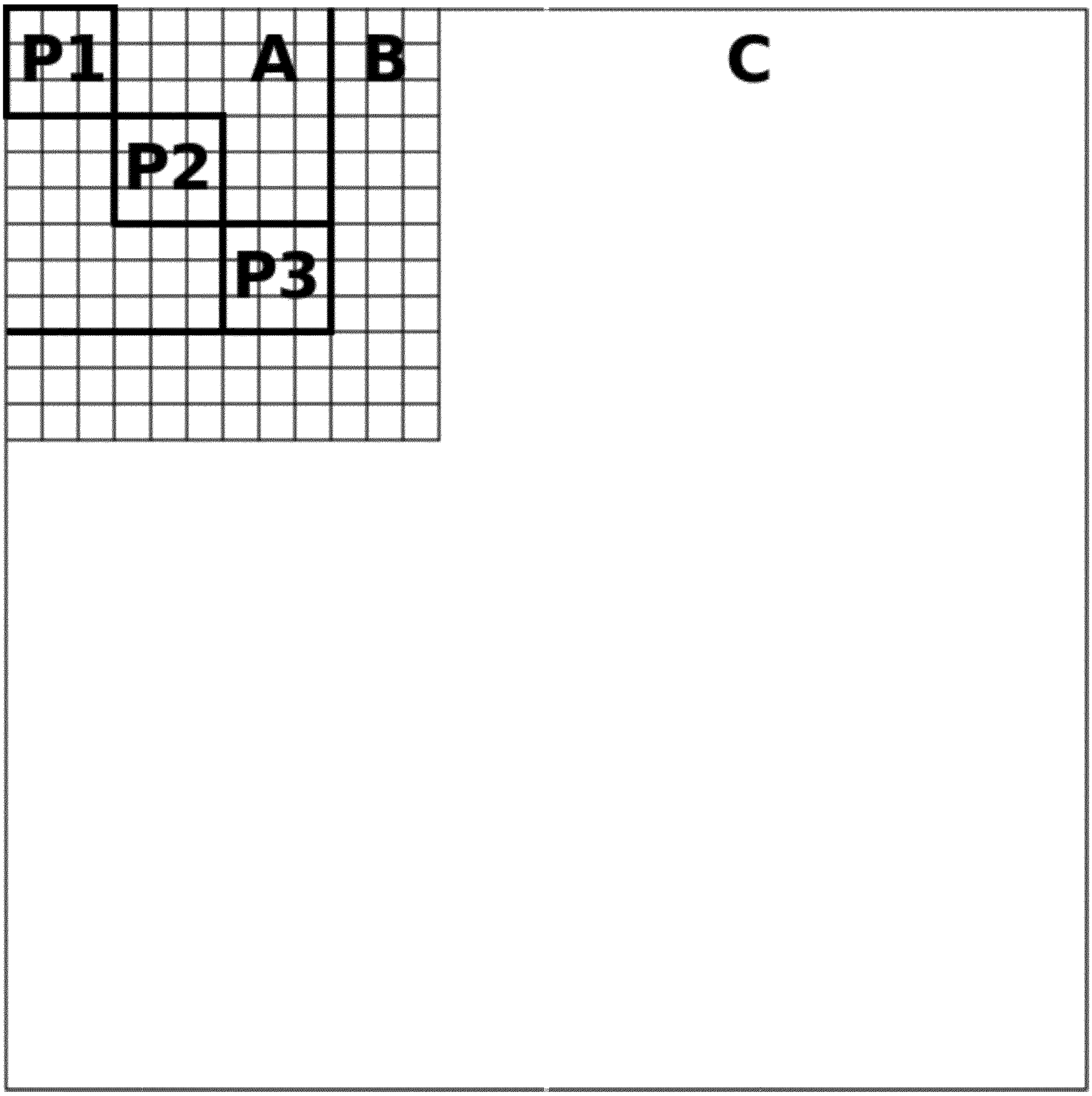


Figure 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016018960 A1
- WO 2018136248 A1
- WO 2018204423 A1
- WO 2011100617 A1
- US 2019085384 A


### Non-patent literature cited in the description


- **BUSCHMANN** ; **BYSTRYKH**. *BMC Bioinformatics*, 2013, vol. 14, 272
- **CONWAY** ; **SLOANE**. *IEEE Trans. Inf. Theor.*, 1986, vol. 32 (3), 337-348
- **HAWKINS et al.** *PNAS*, 2018, vol. 115 (27), E6217-E6226
- **MACCONAILL et al.** *BMC Genomics*, 2018, vol. 19 (1), 30-30